(19) Europäisches Patentamt
European Patent Office
Office européen des brevets

(11) EP 3 736 266 A1

(12) EUROPEAN PATENT APPLICATION
published in accordance with Art. 153(4) EPC

(43) Date of publication:
**11.11.2020 Bulletin 2020/46**

(21) Application number: **18898124.5**

(22) Date of filing: **04.01.2018**

(51) Int Cl.:
***C07D 211/00*** *(2006.01)*

(86) International application number:
**PCT/CN2018/071317**

(87) International publication number:
**WO 2019/134087 (11.07.2019 Gazette 2019/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Peking University Shenzhen Graduate School**
**Guangdong 518055 (CN)**

(72) Inventors:
- **QUAN, Junmin**
**Shenzhen, Guangdong 518055 (CN)**
- **LI, Qinkai**
**Shenzhen, Guangdong 518055 (CN)**
- **ZENG, Xin**
**Shenzhen, Guangdong 518055 (CN)**
- **XU, Zhengshuang**
**Shenzhen, Guangdong 518055 (CN)**
- **YUE, Hong**
**Shenzhen, Guangdong 518055 (CN)**
- **ZHANG, Tingyao**
**Shenzhen, Guangdong 518055 (CN)**
- **FU, Jiamiao**
**Shenzhen, Guangdong 518055 (CN)**
- **HUANG, Xinyi**
**Shenzhen, Guangdong 518055 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) COMPOUND SIMULTANEOUSLY INHIBITING LSD1 AND HDAC TARGETS AND APPLICATION THEREOF

(57) A compound having a general structural formula as shown in Formula I: X-AB-Y (Formula I); in the above Formula I, X is selected from any one of -CO2H, -CONHZ, -CH=CH-$CO_2$H, -CH=CH-CONHZ, wherein Z is selected from any one of substituted or unsubstituted C1-C12 alkyl, substituted or unsubstituted aryl, and hydroxyl; Y = -NR1R2, wherein NR1R2 is a substituted or unsubstituted 3- to 9-membered nitrogen-containing heterocycloalkyl; A and B are each independently selected from substituted or unsubstituted phenylene, substituted or unsubstituted azaphenylene. The compound or corresponding pharmaceutical salt thereof can inhibit LSD1 and HDAC target proteins at the same time, thus inhibit the proliferation of many kinds of tumor cells and have good antitumor effect.

EP 3 736 266 A1

## Description

### Technical Field

The present invention belongs to the technical field of medicines, and particularly relates to a compound for simultaneously inhibiting LSD1 and HDAC targets and an application thereof.

### Background Art

Histone modifications include methylation, acetylation, phosphorylation, ubiquitination, and the like. Histone methylation occurs at arginine and lysine residues of histones H3 and H4, and can occur 1-2 times (arginine) or 1-3 times (lysine). It is regulated by histone methyltransferase (HMT) and histone demethylase (HDM) and characterized by transcriptional activation or gene silencing. Histone acetylation process is jointly regulated by histone acetyltransferase (HAT) and histone deacetylase (HDAC). Most histones in the transcriptional activation region show highly acetylated state, while deacetylated state usually shows gene silencing. Abnormal histone modification is also an important cause of cancer development.

The action mechanism of histone deacetylases (HDACs) is to remove the acetyl group of nucleosome histone lysine residue, reduce the space between nucleosome and DNA, at the same time, make histone return to positive electricity, enhance the electrostatic interaction between nucleosome and DNA, make DNA tightly entangled on nucleosome, thus hinder the binding of transcription factor to DNA and inhibit the process of transcription. Histone acetylation is closely related to the development of cancer and the expression of oncogenes and tumor suppressor genes. Many cancers show abnormal expression of histone acetyltransferase and deacetylase, usually characterized by overexpression or activation of HDACs, which inhibits the expression of specific genes. Studies have shown that core histone H4 in human cancer cells usually exhibits low acetylation at the overall level. Moreover, in addition to regulating the acetylation level of histone, HDACs can also bind and regulate many other protein factors, including transcription factors p53, E2F1, nuclear factor NF-$\kappa$B, protein factor $\alpha$-tubulin, Ku70, heat shock protein Hsp90, etc., and can target them for deacetylation to affect their activity.

Studies have shown that histone deacetylation inhibitor (HDACi) can increase the level of histone acetylation in cancer cells, activate the expression of tumor suppressor genes and induce cancer cell apoptosis by inhibiting the effect of HDACs. Therefore, HDACs have become a hot spot in the field of cancer drug research and development. Like most anticancer drugs, HDACi can induce biological and morphological apoptosis in cancer cells. Animal experiments and clinical studies have shown that concentrations of HDACi that exhibit anti-cancer activity are less toxic to the host, dose-limiting toxicities generally include thrombocytopenia, nausea and fatigue, and in most cases these side effects are clinically controllable. HDACi can induce apoptosis by blocking cell cycle, and has anti-angiogenic, anti-spreading and immunoregulatory activities. HDACi may act directly on cancer cells to enhance their immunogenicity, as well as increase the activity of immune cells and promote cytokine production, thereby enhancing antitumor immunity. Methylation/demethylation of histones is also an important research direction in epigenetics. With the discovery of the first histone lysine demethylase (LSD1), the view that histone methylation process is irreversible is revised, and laid the foundation for the discovery of more histone demethylases and function research thereof. The increase in the number of histone demethylases highlights the nature of dynamic regulation of histone methylation, which is a key chromatin modification involved in genome and gene regulation in eukaryotes. It plays an extensive role in cell proliferation, adipogenesis, spermatogenesis, chromosome segregation and embryonic development. Moreover, LSD1 can also promote tumor growth by inhibiting the activity of tumor suppressor p53. A series of studies have shown the specific biological effects of these enzymes and their potential links to human diseases.

LSD1 plays an indispensable role in the development and differentiation of mammals, which can regulate hormone levels, affect the proliferation and differentiation of hematopoietic cells and inhibit energy consumption and lipolysis. The overexpression of LSD1 is closely related to the occurrence and development of prostate cancer, breast cancer, colon cancer, neuroblastoma, non-small cell lung cancer and bladder cancer, and plays a significant role in the self-renewal and differentiation of stem cells and tumor stem cells. Inhibition of the expression or function of LSD1 has an important role in the treatment of cancer. LSD1, a new tumor drug target, has broad prospects for research, development and clinical application.

Histone demethylase LSD1 and histone deacetylase HDACs are key proteins of transcriptional suppressor complexes such as CoREST and NuRD. They extensively regulate the activation and inhibition of gene transcription by synergism, which are highly related to the occurrence and development of many kinds of tumors. Histone demethylase and histone deacetylase are highly expressed in a variety of tumor cells and have a variety of biological functions, including promoting tumor proliferation, promoting fat synthesis, inhibiting energy metabolism, inhibiting lipolysis, regulating cell differentiation and so on. Therefore, LSD1 and HDACs can be used as targets of antitumor drugs, and the research and discovery of their inhibitors are of great significance for the research and development of new antitumor

drugs. At present, the main research on inhibitors is that single targeting LSD1 or HDACs, does not make full use of the synergistic effect of LSD1 and HDACs. It has been found in studies that the combination administration of LSD1 and HDACs small molecular inhibitors has a significant synergistic effect on the inhibitory activity of a variety of tumors, but the combination brings great challenges to clinical research because of the different pharmacokinetic properties of the combination drugs and the possible drug-drug interaction. Therefore, the study of bifunctional small molecule inhibitors targeting LSD1/HDACs at the same time has become a frontier research direction in this field.

## Technical Problem

The present invention aims to overcome the defects in the prior art, provides a compound for simultaneously inhibiting LSD1 and HDAC targets and an application thereof, and aims to improve the synergistic effect of an existing epigenetic regulation drug in tumor treatment.

## Technical Solution

In order to achieve the purpose, the present invention adopts the following technical solution: One aspect of the present invention provides a compound having a general structural formula shown in Formula I:

X-AB-Y Formula I

In the above Formula I,

- X is selected from any one of $-CO_2H$, -CONHZ, $-CH=CH-CO_2H$, -CH=CH-CONHZ, wherein Z is selected from any one of substituted or unsubstituted $C_1$-$C_{12}$ alkyl, substituted or unsubstituted aryl, and hydroxyl;
- Y = $-NR_1R_2$, wherein $NR_1R_2$ is a substituted or unsubstituted 3- to 9-membered nitrogen-containing heterocycloalkyl;
- A and B are each independently selected from substituted or unsubstituted phenylene, substituted or unsubstituted azaphenylene.

Accordingly, a pharmaceutically acceptable salt of one of the above compounds of the present invention.

Accordingly, a pharmaceutical composition comprising a compound of the present invention as described above and a pharmaceutically acceptable carrier.

Finally, the present invention provides the use of a compound of the present invention as described above, or a pharmaceutically acceptable salt thereof in preparation of a drug for the treatment and/or prevention of a tumor or cancer associated with LSD1 and/or HDAC.

## Advantageous Effects

A computer-aided design method is utilized in the present invention in combination with the experimental detection method, based on the crystal structure of the active pocket of LSD1 and HDAC1, to make full use of the common structure of the two kinds of protein active pocket to design and synthesize a series of biaryl small molecular compounds which can better inhibit LSD1 and HDACs at the same time. The compound or corresponding medicinal salt thereof can inhibit both LSD1 and HDAC target proteins, thus inhibiting the proliferation of tumor cells, showing a synergistic inhibitory effect in a variety of tumor cells, and can be used in the prevention and treatment of tumors or cancers associated with histone acetylation and methylation abnormalities.

## Embodiments of the Invention

In order to make the technical problems to be solved, the technical scheme and the beneficial effect of the present invention clearer, the present invention is further explained in detail in combination with the following embodiments. It is to be understood that the specific embodiments described herein are merely illustrative of the present invention and are not intended to be limiting thereof.

The compounds and derivatives thereof referred to in the embodiments of the present invention are named according to the IUPAC (International Union of Pure and Applied Chemistry) or CAS (Chemical Abstracts Service) nomenclature system. Thus, the compound groups specifically referred to in the embodiments of the present invention are illustrated and described as follows: "Alkyl" means a straight or branched, monovalent, saturated aliphatic chain including, but not limited to, groups such as methyl, ethyl, propyl, isopropyl, butyl, isobutyl, pentyl, isopentyl, hexyl, and the like.

"Heteroalkyl" refers to a straight or branched, monovalent, saturated aliphatic chain linked to at least one

heteroatom, for example, but not limited to, methylaminoethyl or other similar groups. "Alkenyl" means a straight or branched chain hydrocarbon having one or more double bonds and including, but not limited to, groups such as ethenyl, propenyl, and the like.

"Heteroalkenyl" means a straight or branched chain hydrocarbon having one or more double bonds linked to at least one heteroatom and including, but not limited to, groups such as vinylaminoethyl or the like.

"Alkynyl" refers to straight or branched chain hydrocarbons having one or more triple bonds and including, but not limited to, groups such as ethynyl, propynyl, and the like.

"Heteroalkynyl" means a straight or branched chain hydrocarbon having one or more triple bonds attached to at least one heteroatom and including, but not limited to, groups such as ethynyl, propynyl, and the like.

"Aryl" means a cyclic aromatic hydrocarbon including, but not limited to, groups such as phenyl, naphthyl, anthryl, phenanthryl, and the like.

"Heteroaryl" means a monocyclic or polycyclic or fused ring aromatic hydrocarbon in which one or more carbon atoms have been substituted with a heteroatom such as nitrogen, oxygen, or sulfur. If a heteroaryl contains more than one heteroatom, the heteroatoms may be the same or different. Heteroaryls include, but are not limited to, groups such as benzofuryl, benzothienyl, benzimidazolyl, benzoxazolyl, benzothiazolyl, benzopyranyl, furyl, imidazolyl, indazolyl, indolizinyl, indolyl, isobenzofuryl, isoindolyl, isoquinolinyl, isothiazolyl, isoxazolyl, naphthyridinyl, oxadiazolyl, oxazinyl, oxazoyl, phthalazinyl, pteridinyl, purinyl, pyranyl, pyrazinyl, pyrazolyl, pyridazinyl, pyrido[3,4-b]indolyl, pyridyl, pyrimidinyl, pyrrolyl, quinolizinyl, quinolinyl, quinoxalinyl, thiadiazolyl, thiatriazolyl, thiazolyl, thienyl, triazinyl, triazolyl, xanthenyl and the like.

"Cycloalkyl" refers to a saturated monocyclic or polycyclic alkyl group, possibly fused to an aromatic hydrocarbon group. Cycloalkyls include, but are not limited to, groups such as cyclopropyl, cyclobutyl, cyclopentyl, cyclohexyl, cycloheptyl, indanyl, tetrahydronaphthyl, and the like.

"Heterocycloalkyl" means a saturated monocyclic or polycyclic alkyl group which may be fused to an aromatic hydrocarbon group in which at least one carbon atom has been substituted by a heteroatom such as nitrogen, oxygen or sulfur. If a heterocycloalkyl contains more than one heteroatom, the heteroatoms may be the same or different. Heterocycloalkyl groups include, but are not limited to, groups such as azabicycloheptanyl, azetidinyl, indolinyl, morpholinyl, piperazinyl, piperidinyl, pyrrolidinyl, tetrahydrofuranyl, tetrahydroquinolinyl, tetrahydroindazolyl, tetrahydroindolyl, tetrahydroisoquinolinyl, tetrahydropyranyl, tetrahydroquinoxalinyl, tetrahydrothiopyranyl, thiazolidinyl, thiomorpholinyl, thioxanthenyl, thioxanyl, and the like.

"Cycloalkenyl" refers to an unsaturated, monocyclic or polycyclic alkenyl group having one or more double bonds, which may be fused to an aromatic hydrocarbon group, including, but not limited to, cycloethenyl, cyclopropenyl, or other similar groups.

"Heterocycloalkenyl" refers to an unsaturated, monocyclic or polycyclic alkenyl group having one or more double bonds, which may be fused to an aromatic hydrocarbon group, in which at least one carbon atom is substituted by a heteroatom such as nitrogen, oxygen or sulfur. If a heterocycloalkyl contains more than one heteroatom, the heteroatoms may be the same or different.

"Cycloalkynyl" refers to an unsaturated, monocyclic or polycyclic alkynyl group having one or more triple bonds, which may be fused to an aromatic hydrocarbon group, including, but not limited to, cycloethynyl, cyclopropynyl, or other similar groups.

"Heterocycloalkynyl" refers to an unsaturated, monocyclic or polycyclic alkynyl group having one or more triple bonds, which may be fused to an aromatic hydrocarbon group, in which at least one carbon atom is substituted by a heteroatom such as nitrogen, oxygen or sulfur. If a heterocycloalkyl contains more than one heteroatom, the heteroatoms may be the same or different.

It should be noted that in embodiments of the present invention, the symbol 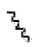 represents the point of attachment of a moiety in a compound to the remainder of the compound.

In one aspect, embodiments of the present invention provide a compound having a general structural formula shown in Formula I:

X-AB-Y    Formula I

In the above Formula I,

X is selected from any one of $-CO_2H$, -CONHZ, $-CH=CH-CO_2H$, -CH=CH-CONHZ, wherein Z is selected from any one of substituted or unsubstituted $C_1$-$C_{12}$ alkyl, substituted or unsubstituted aryl, and hydroxyl;
$Y = -NR_1R_2$, wherein $NR_1R_2$ is a substituted or unsubstituted 3- to 9-membered nitrogen-containing heterocycloalkyl;
A and B are each independently selected from substituted or unsubstituted phenylene, substituted or unsubstituted azaphenylene.

Further, in the above Formula I, A and B are each independently selected from: any one of

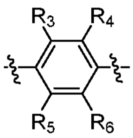 、 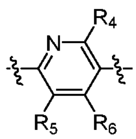 、 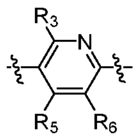 、 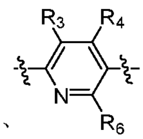 、 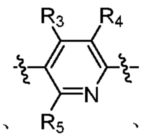 、 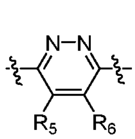 、 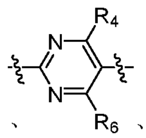 、

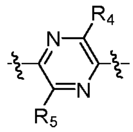 、 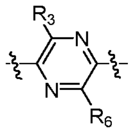 、 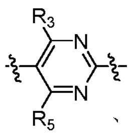 、 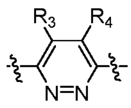 ;

Wherein $R_3$, $R_4$, $R_5$ and $R_6$ are each independently selected from any one of substituted or unsubstituted $C_1$-$C_{12}$ alkyl, substituted or unsubstituted $C_1$-$C_{12}$ heteroalkyl, substituted or unsubstituted $C_3$-$C_{12}$ cycloalkyl, substituted or unsubstituted $C_3$-$C_{12}$ heterocycloalkyl, substituted or unsubstituted $C_2$-$C_{12}$ alkenyl, substituted or unsubstituted $C_2$-$C_{12}$ heteroalkenyl, substituted or unsubstituted $C_3$-$C_{12}$ cycloalkenyl, substituted or unsubstituted $C_3$-$C_{12}$ heterocycloalkenyl, substituted or unsubstituted $C_2$-$C_{12}$ alkynyl, substituted or unsubstituted $C_2$-$C_{12}$ heteroalkynyl, substituted or unsubstituted $C_3$-$C_{12}$ cycloalkynyl, substituted or unsubstituted $C_3$-$C_{12}$ heterocycloalkynyl, substituted or unsubstituted aryl, substituted or unsubstituted heteroaryl, aryl ($C_1$-$C_{12}$) alkyl, heteroaryl ($C_1$-$C_{12}$) alkyl, $C_2$-$C_{12}$ alkenyl ($C_1$-$C_{12}$) alkyl, $C_2$-$C_{12}$ alkynyl ($C_1$-$C_{12}$) alkyl, hydroxyl, amino, nitro, sulfo, halogen and hydrogen atom.

More preferably, $R_3$, $R_4$, $R_5$ and $R_6$ are each independently selected from any one of substituted or unsubstituted $C_1$-$C_6$ alkyl, substituted or unsubstituted $C_1$-$C_6$ heteroalkyl, substituted or unsubstituted $C_3$-$C_6$ cycloalkyl, substituted or unsubstituted $C_3$-$C_6$ heterocycloalkyl, substituted or unsubstituted $C_2$-$C_6$ alkenyl, substituted or unsubstituted $C_2$-$C_6$ heteroalkenyl, substituted or unsubstituted $C_3$-$C_6$ cycloalkenyl, substituted or unsubstituted $C_3$-$C_6$ heterocycloalkenyl, substituted or unsubstituted $C_2$-$C_6$ alkynyl, substituted or unsubstituted $C_2$-$C_6$ heteroalkynyl, substituted or unsubstituted $C_3$-$C_6$ cycloalkynyl, substituted or unsubstituted $C_3$-$C_6$ heterocycloalkynyl, substituted or unsubstituted aryl, substituted or unsubstituted heteroaryl, aryl ($C_1$-$C_6$) alkyl, heteroaryl ($C_1$-$C_6$) alkyl, $C_2$-$C_6$ alkenyl ($C_1$-$C_6$) alkyl, $C_2$-$C_6$ alkynyl ($C_1$-$C_6$) alkyl. More preferably, the $R_3$, $R_4$, $R_5$ and $R_6$ are each independently selected from substituted or unsubstituted $C_1$-$C_4$ alkyl.

Further, in the above Formula I, the structure of -AB- is selected from: any one of

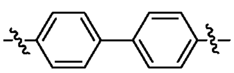 、 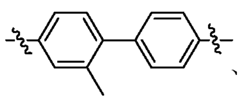 、 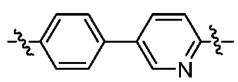 、 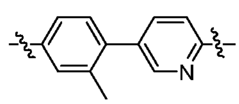 、

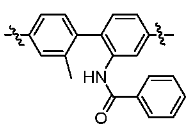 、 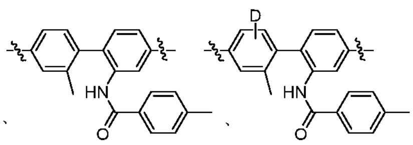 ;

wherein D is halogen (e.g. fluorine, chlorine, bromine, iodine).

Further, in the above Formula I, Z is a substituted or unsubstituted $C_1$-$C_4$ alkyl group or a hydroxyl group.

Further, in the above Formula I, the structure of Z is

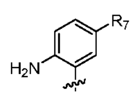

or hydroxy; wherein $R_7$ is selected from any one of substituted or unsubstituted $C_1$-$C_{12}$ alkyl, substituted or unsubstituted $C_1$-$C_{12}$ heteroalkyl, substituted or unsubstituted $C_3$-$C_{12}$ cycloalkyl, substituted or unsubstituted $C_3$-$C_{12}$ heterocycloalkyl, substituted or unsubstituted $C_2$-$C_{12}$ alkenyl, substituted or unsubstituted $C_2$-$C_{12}$ heteroalkenyl, substituted or unsubstituted $C_3$-$C_{12}$ cycloalkenyl, substituted or unsubstituted $C_3$-$C_{12}$ heterocycloalkenyl, substituted or unsubstituted $C_2$-$C_{12}$ alkynyl, substituted or unsubstituted $C_2$-$C_{12}$ heteroalkynyl, substituted or unsubstituted $C_3$-$C_{12}$ cycloalkynyl, substituted or unsubstituted $C_3$-$C_{12}$ heterocycloalkynyl, substituted or unsubstituted aryl, substituted or unsubstituted heteroaryl,

aryl ($C_1$-$C_{12}$) alkyl, heteroaryl ($C_1$-$C_{12}$) alkyl, $C_2$-$C_{12}$ alkenyl ($C_1$-$C_{12}$) alkyl, $C_2$-$C_{12}$ alkynyl ($C_1$-$C_{12}$) alkyl, hydroxyl, amino, nitro, sulfo, halogen and hydrogen atom.

Further, in the above Formula I, Y is a substituted or unsubstituted 5- or 6-membered nitrogen-containing heterocyclohydrocarbyl. More preferably, Y is selected from: any one of

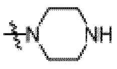, 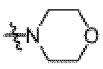, 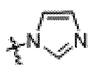, 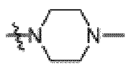, 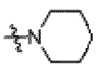.

In particular, in a preferred embodiment of the present invention, the compound is selected from any one of

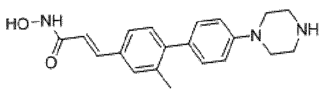, 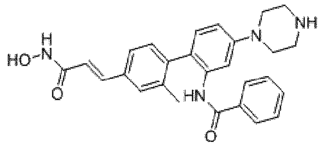, 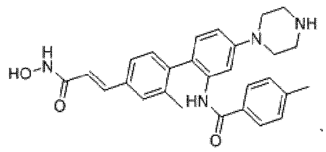,

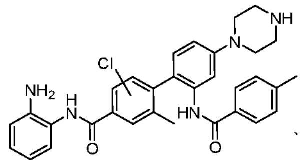, 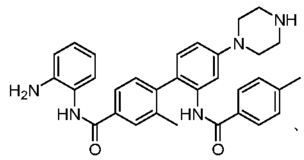, 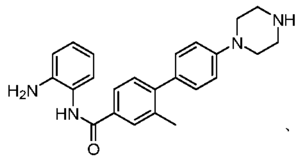,

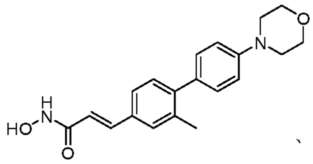, 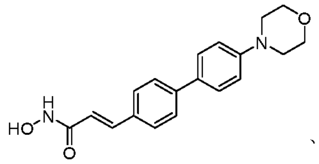, 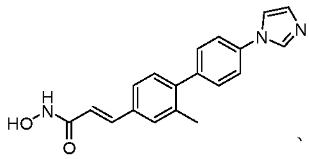,

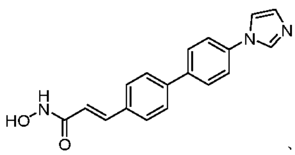, 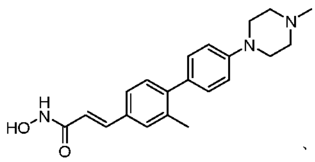, 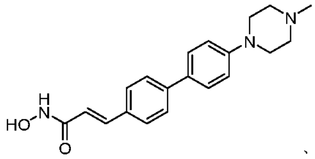,

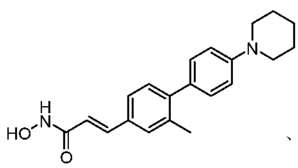, 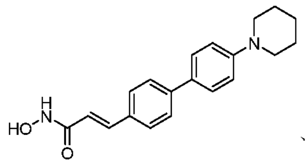, 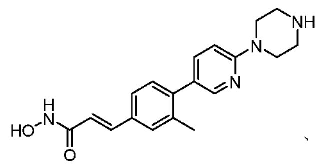,

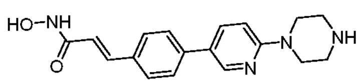.

In the embodiments of this specification, the preferred 16 compounds are designated as compounds of the ZZY series (i.e., ZZY-001 to ZZY-016 in turn). Accordingly, in an embodiment of the present invention, a pharmaceutically acceptable salt of the above compound of an embodiment of the present invention is also provided.

Accordingly, in an embodiment of the present invention, a pharmaceutical composition comprising a compound of the present invention as described above and a pharmaceutically acceptable carrier is also provided. In particular, auxiliary materials such as diluents, adhesives, absorbents, disintegrants, dispersants, wetting agents, co-solvents, buffers, surface activators, etc., needed in the preparation of pharmaceutical compositions are included. The pharmaceutical composition can be used for preventing and treating tumors or cancers related to histone acetylation and methylation abnormality. In particular, the pharmaceutical composition further comprises an anticancer drug. The anti-

cancer drug is an LSD1 inhibitor and/or an HDAC inhibitor.

Finally, in an embodiment of the present invention, the use of a compound as described above in an embodiment of the present invention, or a pharmaceutically acceptable salt thereof in preparation of a drug for the treatment and/or prevention of a tumor or cancer associated with LSD1 and/or HDAC is also provided. In particular, the tumor or cancer is selected from: at least one of brain cancer, glioblastoma, leukemia, Bristlecone syndrome, Cowden's Disease, cerebellar dysplastic gangliocytoma, breast cancer, inflammatory breast cancer, Wilm's tumor, Ewing's sarcoma, rhabdomyosarcoma, ependymoma, medulloblastoma, colon cancer, head and neck cancer, kidney cancer, lung cancer, liver cancer, melanoma, kidney cancer, ovarian cancer, pancreatic cancer, prostate cancer, sarcoma, osteosarcoma, giant cell tumor of the bone and thyroid.

In embodiments of the present invention, the general formula for the synthesis of compounds of the ZZY series is shown below in the reverse synthesis analysis of ZZY-001. The synthesis of ZZY-001 can be carried out by HWE reaction of intermediates 1 and 2 to complete the carbon chain extension, wherein the hydroxamic acid at the carboxylic acid end can be realized in the form of an amide bond, and the aldehyde group of intermediate 2 can be conveniently oxidized into carboxylic acid and derivatized. The key to the synthesis of intermediate 2 is the biaryl structure, which can be prepared via intermediates 3 and 4 using Suzuki coupling reactions.

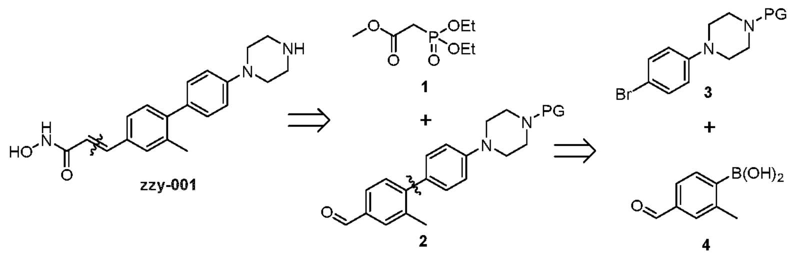
zzy-001 1 + 2 3 + 4

The present invention has been successively tested many times, and now a part of the test results are used as a reference to further describe the present invention in detail, and a detailed description will be given below in conjunction with specific embodiments.

**Embodiment 1**

Wherein the synthesis of the intermediate 3 can be completed by the following several routes according to different requirements of the substrate structure:

Route I: N-alkylation reaction as a critical step

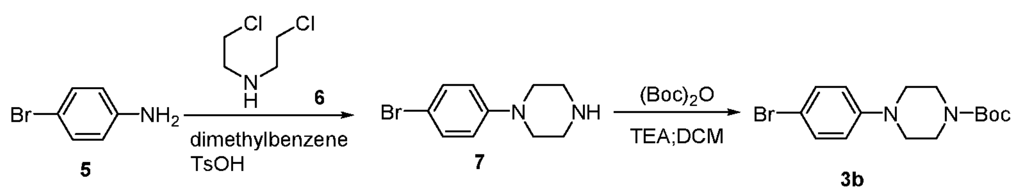

5 7 3b

P-bromoaniline 5 (1.5 eq.), bis (2-chloroethyl) amine hydrochloride 6 (1 eq.) and a catalytic amount of p-toluenesulfonic acid were dissolved in xylene and refluxed. After the end of the TLC tracking reaction, cooled to room temperature and filtered the collected products. The filter cake was washed with water, and then transferred into a round bottom flask, water was added to heat up and reflux to dissolve and clarify, cooled to room temperature again to precipitate solids. Product 7 was obtained by filtering.

Compound 7 (1 eq.) was dissolved in dichloromethane, triethylamine (3 eq.) and Boc anhydride (2 eq.) were added successively and stirred at room temperature overnight. After the reaction was finished, water was added to quench the reaction. Extracted three times with dichloromethane, combined with the organic phases, washed with saturated brine, dried and concentrated. 3b was obtained by column chromatography.

Route II: obtaining through bromo-reduction route

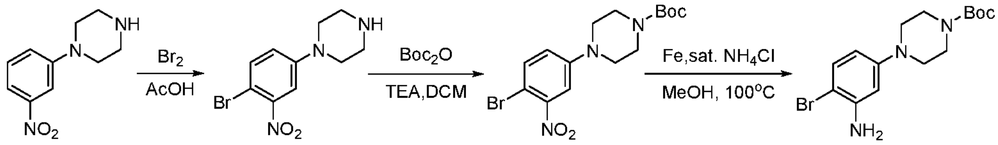


**8** **9** **10** **3a**

1-(3-nitrophenyl) piperazine 8 (1 eq.) was dissolved in acetic acid and bromine (1.5 eq.) was added dropwise and stirred at 75°C overnight. After the reaction was finished, the product was collected by filtration, the filter cake was washed with n-hexane, then the filter cake was transferred to a round bottom flask, methanol was added, residual bromine was removed by rotary evaporation, and the product 9 was obtained by vacuum drying. Compound 9 (1 eq.) was dissolved in dichloromethane, triethylamine (3 eq.) and Boc anhydride (2 eq.) were added successively and stirred at room temperature overnight. After the reaction was finished, water was added to quench the reaction, and then extracted with dichloromethane for three times, combined with the organic phases, washed with saturated brine, dried and concentrated. Product 10 was obtained by column chromatography. A round bottom flask was charged with iron powder (3 eq.) and saturated ammonium chloride solution and heated up to 100°C. Compound 10 (1 eq.) was dissolved in methanol and added dropwise into a round bottom flask. Stirring at reflux for 2 hours, cooling to room temperature after the reaction was finished, water was added to quench the reaction, then extracted with dichloromethane for three times, combined with the organic phases, washed with saturated brine, dried and concentrated. 3a was obtained by column chromatography.

Route III: aromatic amination as a key step

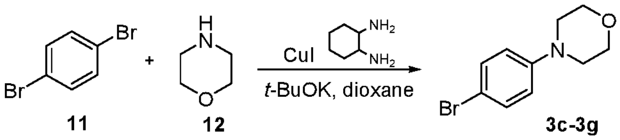


**11** **12** **3c-3g**

Morpholine 12 (1.1 eq.), p-dibromobenzene 11 (1 eq.) and 2% cuprous iodide were dissolved in 1,4-dioxane and potassium tert-butoxide (2 eq.) and 10% cyclohexanediamine were added and stirred at reflux temperature overnight. The reaction was quenched with saturated ammonium chloride, then extracted with ethyl acetate for three times and combined with organic phases, washed with saturated brine, dried and concentrated. Compound 3c was obtained by column chromatography. 3d - 3g can be prepared in the same manner.

The structure and analytical data of intermediate 3 are as follows:

**3a:** 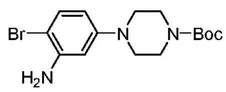

$^1$H NMR (500 MHz, MeOH-*d4*) δ 7.18-7.16 (*d,* 1H), 6.46-6.44 (*d,* 1H), 6.27-6.24 (*m,* 1H), 3.53 (*sbr,* 4H), 3.06-3.03 (*m,* 4H), 1.49 (*s*, 9H) ppm; ESI-MS obsd 356.0965, calcd 356.0968 [(M + H)$^+$, M = $C_{15}H_{22}BrN_3O_2$].

**3b:** 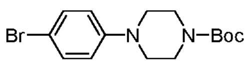

$^1$H NMR (400 MHz, $CDCl_3$) δ 7.36-7.33 (*m,* 2H), 6.80-6.77 (*m,* 2H), 3.58-3.55 (*m,* 4H), 3.11-3.07 (*m*, 4H), 1.48 (*s*, 9H) ppm; ESI-MS obsd 341.0859, calcd341.0859 [(M + H)$^+$, M = $C_{15}H_{21}BrN_2O_2$].

3c: 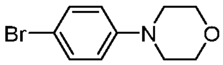

$^1H$ NMR (300 MHz, $CDCl_3$) $\delta$7.48-7.36(*m*, 2H), 6.71-6.52 (*m,* 2H), 3.71 (*t,* 4H), 2.99 (*t,* 4H)ppm;ESI-MSobsd 242.0177, calcd 242.0175 [(M + H)$^+$, M = $C_{10}H_{12}BrNO$].

3d: 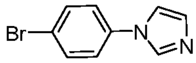

$^1H$ NMR (300 MHz, $CDCl_3$) $\delta$7.26 (*s*, 1H), 7.27 (*s*, 1H),7.36-7.25 (*m,* 2H), 7.67-7.60 (*m,* 2H), 7.87 (*s*, 1H) ppm; ESI-MS obsd 222.9865, calcd 222.9865 [(M + H)$^+$, M = $C_9H_7BrN_2$].

3e: 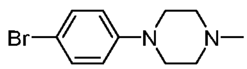

$^1H$ NMR (500 MHz, $CDCl_3$) $\delta$7.34-7.31 *(d,* 2H), 6.79-6.73 *(d,* 2H), 3.26-3.22(*t*, 4H), 2.59-2.51 (*t,* 4H), 2.34 (s, 3H) ppm; ESI-MS obsd 254.0494, calcd 255.0491 [(M + H)$^+$, M = $C_{11}H_{15}BrN_2$].

3f: 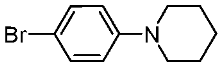

$^1H$ NMR (400 MHz, $CDCl_3$) $\delta$ 7.31-7.27 (*m,* 2H), 6.81-6.77 (*m,* 2H), 3.13-3.08(*m*, 4H), 1.74-1.67 (*m,* 4H), 1.58-1.54 (*m*, 2H)ppm;ESI-MSobsd 240.0383, calcd 240.0382 [(M + H)$^+$, M = $C_{11}H_{14}BrN$].

3g: 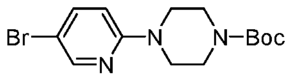

$^1H$ NMR (300 MHz, $CDCl_3$)$\delta$8.20 (*s*, 1H), 7.56-7.54 (*d,* 1H), 6.55-6.54 (*d,* 1H), 3.53-3.50 (*m,* 8H),1.49 (*s*, 9H) ppm; ESI-MS obsd 342, calcd 342 [(M + H)$^+$, M = $C_{14}H_{20}BrN_3O_2$].

**Embodiment 2**

Intermediate 4 can be obtained by using commercially available borate-based compounds, such as intermediate 4a, or obtained by borylation of properly protected and substituted 4-bromobenzaldehyde under transition metal catalysis, for example, the synthesis route of intermediate 4b was as follows:

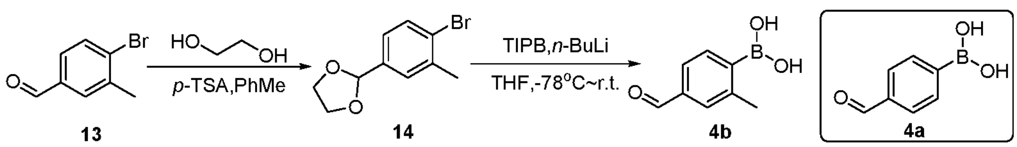

13 14 4b 4a

4-bromo-3-tolualdehyde 13 (1 eq.) was dissolved in toluene, ethylene glycol (4 eq.) and catalytic amount of p-toluenesulfonic acid were added successively, connected with Dean-Stark device, and refluxed for 20 hours. After the reaction was finished, cooled to room temperature, poured the reaction mixture into saturated sodium bicarbonate solution, then extracted with ethyl acetate for three times, combined the organic phase, then washed with saturated salt water, dried and concentrated. Product 14 was obtained by column chromatography. Compound 14 (1 eq.) was dissolved in dry tetrahydrofuran, cooled to -78°C under the protection of nitrogen, n-butyl lithium (1.2 eq.) was added, stirred for half an hour, triisopropyl borate (1.2 eq.) was added to the reaction system, stirred for 1 hour, slowly returned to room temperature and stirred overnight. After the reaction was finished, the reaction was thoroughly quenched by adding 3N hydrochloric acid solution and stirring for 3 hours, then extracted with ethyl acetate for three times, combined with the organic phases, washed with saturated brine, dried and concentrated. 4b was obtained by column chromatography.

Analytical data for intermediate 4b are $^1H$ NMR (500 MHz, DMSO-*d6*)$\delta$ 9.94 (*s*, 1H), 8.27 (*s*, 2H), 7.63-7.61

(*d,* 2H), 7.59-7.58 (*d,* 1H), 2.44 (*s*, 3H); ESI-MS obsd 187.0537, calcd 187.0537 [(M + Na)$^+$, M = $C_8H_9BO_3$].

Synthesis of intermediate 2

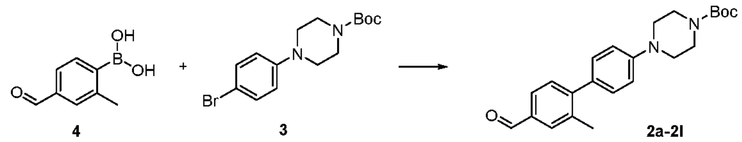

4 3 2a-2l

Intermediate 3 (1 eq.), intermediate 4 (1.3 eq.) and potassium carbonate (4.5 eq.) were dissolved in a mixed solvent of ethylene glycol dimethyl ether and water (V/V = 1:1), and then 5% bis triphenyl-phosphonium palladium dichloride catalyst was added and reacted at 80°C for 6 h under the protection of nitrogen. After completion, cooled to room temperature, the reaction was quenched with water, extracted with ethyl acetate for three times, combined organic phases, washed with saturated brine, dried, and concentrated. Compounds 2a -21 were obtained by column chromatography.

The structure and analytical data for intermediates 2a-21 are as follows:

**2a:** 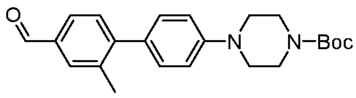

$^1$H NMR(400 MHz, MeOH-*d4*)$\delta$9.96 (*s*, 1H), 7.79 (*s*, 1H), 7.75-7.73 (*m,* 1H), 7.38-7.36 (*d,* 1H), 7.27-7.24 (*m,* 2H), 7.07-7.04 (*m,* 2H), 3.60-3.59 (*m,* 4H), 3.21-3.18 (*m,* 4H), 2.35(*s*, 3H), 1.49 (*s*, 9H) ppm; ESI-MS obsd 381.2172, calcd 381.2173[(M + H)$^+$, M = $C_{23}H_{28}N_2O_3$].

**2b*:** 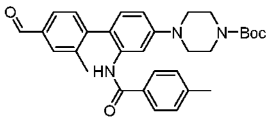

$^1$H NMR (400 MHz, $CDCl_3$)$\delta$10.07 (*s*, 1H), 8.29-8.28 (*d,* 1H), 7.86 (*s*, 1H), 7.83-7.81 (*d,* 1H), 7.77-7.75 (*d,* 1H), 7.46-7.43 (*m,* 2H), 7.36-7.34 (*d,* 2H), 7.17-7.15 (*d,* 2H),7.08-7.05 (*d,* 1H), 6.78-6.76 (*m,* 1H), 3.63-3.60 (*m,* 4H), 3.30-3.28 (*m,* 4H), 2.35 (*s*, 3H), 2.23 (*s*, 3H), 1.50 (*s*, 9H) ppm; ESI-MS obsd 514.2701, calcd 514.2700[(M + H)$^+$, M = $C_{31}H_{36}N_3O_4$].

**2c:** 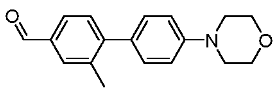

$^1$H NMR (300 MHz, $CDCl_3$)$\delta$10.04-10.02 (*d,* 1H), 7.80-7.73 (*m,* 2H), 7.43-7.28 (*m,* 3H), 7.02-6.98 (*d,* 1H), 6.86-6.83 (*d,* 1H),3.93-3.88 (*m,* 4H), 3.27-3.20 (*m,* 4H), 2.40-2.37 (*d,* 3H)ppm;ESI-MSobsd 282.1491, calcd 282.1489[(M + H)$^+$, M = $C_{18}H_{19}NO_2$].

**2d:** 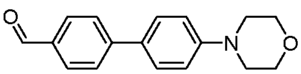

$^1$H NMR (300 MHz, $CDCl_3$)$\delta$10.08-10.04 (*d,* 1H), 7.98-7.93 (*m*,2H), 7.77-7.73 (*m,* 2H), 7.63-6.61(*m,* 1H), 7.17-7.15 (*m,* 1H), 7.04-6.98 (*m,* 2H), 3.93-3.90 (*m,* 4H), 3.28-3.25 (*m*, 4H) ppm; ESI-MS obsd 268.1330, calcd 268.1332[(M + H)$^+$, M = $C_{17}H_{17}NO_2$].

**2e:** 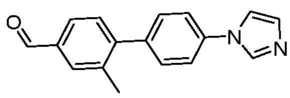

$^1$H NMR (300 MHz, $CDCl_3$)$\delta$8.07 (*s*, 1H), 7.95 (*s*, 1H),7.82-7.78 (*m,* 2H), 7.52-7.39 (*m,* 7H), 2.39 (*s*, 3H) ppm;ESI-MSobsd 263.1180, calcd 263.1179[(M + H)$^+$, M = $C_{17}H_{14}N_2O$].

**2f:**
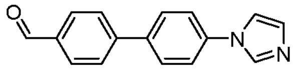

$^1$H NMR (300 MHz, $CDCl_3$)$\delta$10.07 (*s*, 1H), 8.00-7.94 (*m,* 3H), 7.84-7.41 (*m,* 4H), 7.70-7.63 (*m,* 1H), 7.53 (*s*, 1H), 7.50-7.43 (*m,* 1H), 7.35 (*s*, 1H) ppm; ESI-MS obsd 249.1026, calcd 249.1022[(M + H)$^+$, M = $C_{16}H_{12}N_2O$].

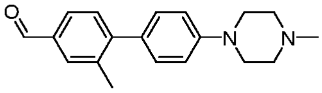
**2g:**

$^1$H NMR (300 MHz, $CDCl_3$)$\delta$10.03-10.01 (*d,* 1H), 7.77-7.72 (*m,* 2H), 7.42-7.38 (*m,* 1H), 7.28-7.25 (*d,* 1H), 7.02-6.95 (*m,* 2H),6.86-6.80 (*m,* 1H), 3.33-3.27 (*m,* 4H), 2.66-2.61 (*m,* 4H), 2.40-2.36 (*m*, 6H)ppm;ESI-MSobsd 295.1806, calcd 295.1805[(M + H)$^+$, M = $C_{19}H_{22}N_2O$].

**2h:**
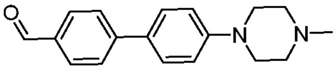

$^1$H NMR (300 MHz, $CDCl_3$)$\delta$10.07-10.03 (*d,* 1H),7.97-7.91 (*m,* 2H), 7.77-7.72 (*m,* 2H), 7.61-7.58 (*m,* 1H), 7.41-7.34 (*m,* 1H),7.18-7.09 (*m,* 1H), 7.04-7.01 (*m,* 1H), 3.35-3.31 (*m,* 4H), 2.67-2.62 (*m,* 4H), 2.40 (*s*, 3H) ppm; ESI-MS obsd 281.1651, calcd 281.1648[(M + H)$^+$, M = $C_{18}H_{20}N_2O$].

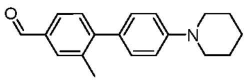
**2i:**

$^1$H NMR (300 MHz, $CDCl_3$)$\delta$10.04-10.02 (*d,* 1H), 7.79-7.73 (*m,* 2H), 7.44-7.40 (*m,* 1H), 7.36-7.31 (*m,* 1H), 7.03-6.97 (*m,* 2H),6.88-6.77 (*d,* 1H), 3.28-3.20 (*m,* 4H), 2.41-2.38 (*d,* 3H), 1.80-1.73 (*m,* 4H), 1.65-1.61 (*m,* 2H) ppm;ESI-MSobsd 280.1695, calcd 280.1696[(M + H)$^+$, M = $C_{19}H_{21}NO$].

**2j:**
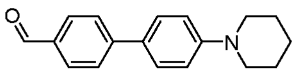

$^1$H NMR (300 MHz, $CDCl_3$)$\delta$10.07-10.03 (*d,* 1H), 7.97-7.91 (*m,* 2H), 7.78-7.73 (*m,* 2H), 7.61-7.58 (*d,* 1H), 7.40--7.18 (*m,* 1H), 7.11-7.01 (*m,* 2H), 3.30-3.24 (*m,* 4H), 1.78-1.72 (*m,* 4H), 1.66-1.63 (*m*, 2H) ppm;ESI-MSobsd 266.1541, calcd 266.1539[(M + H)$^+$, M = $C_{18}H_{19}NO$].

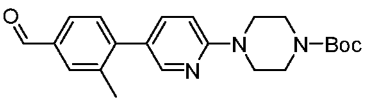
**2k:**

$^1$H NMR (300 MHz, $CDCl_3$)$\delta$10.02 (*s*, 1H), 8.21 (*s*, 1H), 7.79-7.74 (*m,* 2H), 7.54-7.51 (*d,* 1H),7.39-7.36 (*d,* 1H), 6.75-6.72 (*d,* 1H), 3.61 (*s*, 8H),2.40 (*s*, 3H), 1.51 (*s*, 9H) ppm;ESI-MSobsd 382.2129, calcd 382.2125[(M + H)$^+$, M = $C_{22}H_{27}N_3O_3$].

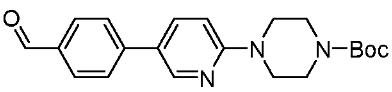
**2l:**

$^1$H NMR (300 MHz, $CDCl_3$)$\delta$10.01 (*s*, 1H), 8.51 (*s*, 1H), 7.93-7.91 (d, 2H), 7.79-7.76 (*d,* 1H),7.68-7.66 (*d,* 2H), 6.73-6.71 (*d,* 1H), 3.63-3.56 (*m,* 8H),1.49 (*s*, 9H) ppm;ESI-MSobsd 368.1970, calcd 368.1969[(M + H)$^+$, M = $C_{21}H_{25}N_3O_3$].

Wherein the preparation of intermediate 2b still requires a one-step amidation reaction after Suzuki coupling, as follows:

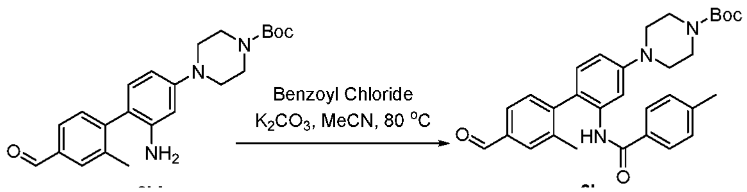


The product 2b' (1 eq.) of the Suzuki reaction was dissolved in acetonitrile, potassium carbonate (2 eq.) was added, cooled to 0°C and p-methylbenzoyl chloride (1.1 eq.) was slowly added dropwise, then heated up to 80°C and stirred for half an hour to finish the reaction. Cooled to room temperature, and the reaction was quenched with water, then extracted with ethyl acetate for three times, combined organic phases, washed with saturated brine, dried, and concentrated. Compound 2b was obtained by column chromatography.

**Embodiment 3**

Trimethyl phosphonoacetate (1.1 eq.) was dissolved in dry tetrahydrofuran, cooled to -20°C, potassium hexamethyldisilazide (1.1 eq.) in tetrahydrofuran (1.0 M) was added dropwise, stirred for 20-30 min, then intermediate 2 (1 eq.) in tetrahydrofuran was added dropwise, stirred for 1 h and the reaction was finished. The reaction was quenched with water, then extracted with ethyl acetate for three times and combined with organic phases, washed with saturated brine, dried and concentrated. Compound 15a was obtained by column chromatography. In this way it was also possible to prepare 15b -15c and 15g -15p, according to the following formula:

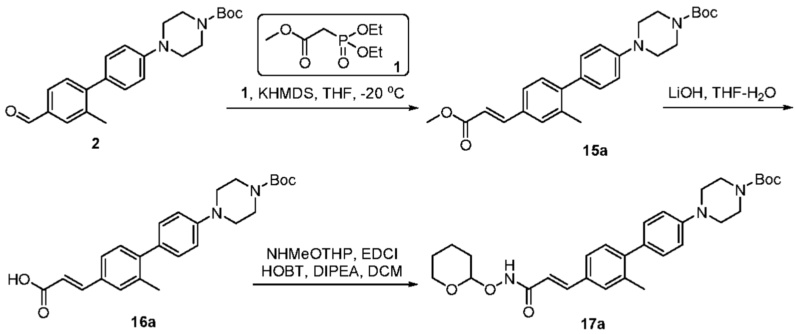


The structure and analytical data of intermediate 15 are as follows:

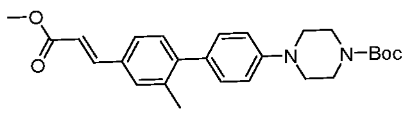

**15a:**

$^1H$ NMR(500 MHz, $CDCl_3$)$\delta$7.71-7.68 (*d,* 1H), 7.41-7.40 (*m,* 2H), 7.25-7.23 (*d,* 3H), 7.00-6.96 (*d,* 2H), 6.46-6.43 (*d,* 1H), 3.81 (*s*, 3H), 3.61-3.59 (*m,* 4H), 3.21-3.20 (*m,* 4H), 2.31 (*s*, 3H), 1.49 (*s*, 9H)ppm;ESI-MSobsd 437.2442, calcd 437.2435 [(M + H)$^+$, M = $C_{26}H_{32}N_2O_4$].

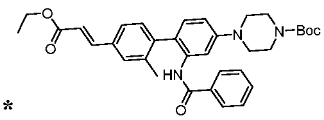

**15b***

$^1H$ NMR (500 MHz, $CDCl_3$)$\delta$8.32 (*sbr,* 1H), 7.73-7.70 (*d,* 1H), 7.59 (*s*, 1H), 7.51 (*s*, 1H), 7.49-7.45 (*m,* 4H), 7.37-7.34 (*m,* 2H), 7.29-7.27 (*d,* 1H), 7.08-7.06 (*d,* 1H), 6.77-6.75 (*m,* 1H), 6.51-6.48 (*d,* 1H), 4.31-4.27 (*m,* 2H), 3.60 (*s*, 4H), 3.28 (*s*, 4H), 2.17 (*s*, 3H), 1.50 (*s*, 9H), 1.37-1.34 (*m,* 3H) ppm; ESI-MS obsd 570.2964, calcd 570.2962[(M + H)$^+$, M = $C_{34}H_{39}N_3O_5$].

**15c*:** 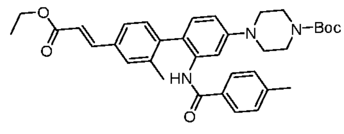

$^1$H NMR (300 MHz, $CDCl_3$)$\delta$8.34 (*sbr,* 1H), 7.77-7.71 (*d,* 1H), 7.59 (*s*, 1H), 7.53-7.49 (*d,* 2H), 7.39-7.37 (*d,* 2H), 7.31 (*s*, 1H), 7.19-7.16 (*d,* 2H), 7.10-7.07 (*d,* 1H),6.78-6.75 (*d,* 1H), 6.55-6.49 (*m,* 1H), 4.35-4.28 (*m,* 2H), 3.65-3.61 (*m,* 4H), 3.31-3.28 (*m,* 4H), 2.37 (*s*, 3H), 2.18 (*s*, 3H), 1.52 (*s*, 9H), 1.41-1.36 *(m,* 3H) ppm; ESI-MS obsd 584.3121, calcd 584.3119[(M + H)$^+$, M = $C_{35}H_{41}N_3O_5$].

**15g:** 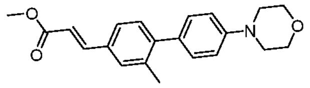

$^1$H NMR (300 MHz, $CDCl_3$)$\delta$7.76-7.70 (*m,* 1H), 7.44-7.40 (*d,* 2H), 7.35-7.27 (*m,* 2H), 7.00-6.83 (*m,* 2H), 6.86-6.83 (*d,* 1H), 6.52-6.45 (*m,* 1H), 3.93-3.88 (*m,* 4H), 3.84-3.83 (*m,* 3H), 3.26-3.20 (*m,* 4H), 2.34-2.30 (*m*, 3H) ppm; ESI-MS obsd 338.1753, calcd 338.1751[(M + H)$^+$, M = $C_{21}H_{23}NO_3$].

**15h:** 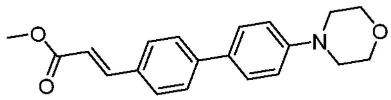

$^1$H NMR (300 MHz, $CDCl_3$)$\delta$7.78 (*s*, 1H), 7.72 (*s*, 1H), 7.62-7.61 (*d*, 4H), 7.57 (*s*, 1H),7.02-7.00 (*d*, 2H), 6.51-6.46 (*d,* 1H), 3.93-3.90 (*m,* 4H), 3.84(*s*, 3H), 3.26-3.23 (*m,* 4H); ESI-MS obsd 324.1596, calcd 324.1594[(M + H)$^+$, M = $C_{20}H_{21}NO_3$].

**15i:** 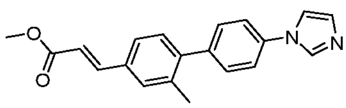

$^1$H NMR (300 MHz, $CDCl_3$)$\delta$7.91 (*s*, 1H),7.72-7.67 (*d,* 1H), 7.44-7.41 (*m,* 6H),7.33 (*s*, 1H), 7.26-7.22 (*m,* 2H), 6.50-6.44 (*d,* 1H), 3.74 (*s*, 3H), 2.39 (*s*, 3H) ppm;ESI-MSobsd 319.1440, calcd 319.1441[(M + H)$^+$, M = $C_{20}H_{18}N_2O_2$].

**15j:** 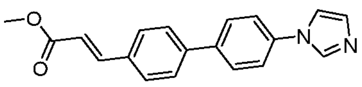

$^1$H NMR (300 MHz, $CDCl_3$)$\delta$7.94 (*s*, 1H), 7.79-7.75 (*d,* 1H), 7.74-7.73 (*d,* 1H), 7.66 (*s*, 4H), 7.50-7.49 (*m,* 4H), 7.36 (*s*, 1H), 6.55-6.49 (*d,* 1H), 3.86-3.85 (*s*, 3H); ESI-MS obsd 305.1287, calcd 305.1285[(M + H)$^+$, M = $C_{19}H_{16}N_2O_2$].

**15k:** 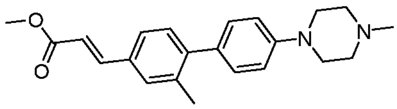

$^1$H NMR (300 MHz, $CDCl_3$)$\delta$7.75-7.69 (*d,* 1H), 7.43-7.40 (*d,* 2H), 7.28-7.24 (*m,* 2H), 7.01-7.00 (*d,* 2H), 6.87-6.81 (*m,* 1H), 6.51-6.44 (*m,* 1H), 3.83 (*s*, 3H), 3.40-3.26 (*m,* 4H), 2.64-2.63 (*m,* 4H), 2.40-2.39 (*m,* 3H), 2.34-2.31 (*m,* 3H) ppm;ESI-MSobsd 351.2065, calcd 351.2067[(M + H)$^+$, M = $C_{22}H_{26}N_2O_2$].

**15l**: 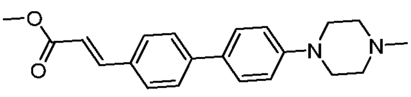

$^1$H NMR (300 MHz, $CDCl_3$)$\delta$7.78-7.71 (*m,* 1H), 7.61-7.57 (*m,* 4H), 7.39-7.34 (*d,* 1H), 7.15-7.10 (*m,* 1H),7.05-6.96 (*m,* 2H), 6.52-6.44 (*m,* 1H), 3.84-3.83 (*m,* 3H), 3.40 (*s*, 4H), 2.66-2.63 (*m,* 4H), 2.40 (*s*, 3H) ppm;ESI-MSobsd 337.1911, calcd 337.1911[(M + H)$^+$, M = $C_{21}H_{24}N_2O_2$].

**15m:** 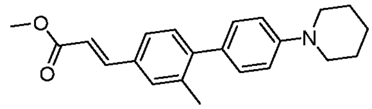

$^1$H NMR (300 MHz, $CDCl_3$)$\delta$7.77-7.71 (*m,* 1H), 7.43-7.41 (*d,* 2H), 7.31-7.27 (*m,* 2H), 7.02-6.95 (*m,* 2H),6.89-6.77 (*m,* 1H), 6.52-6.45 (*m,* 1H), 3.84 (*s*, 3H), 3.27-3.20 (*m,* 4H), 2.54-2.52 (*s*, 3H), 1.80-1.73 (*m,* 4H), 1.67-1.61 (*m,* 2H) ppm;ESI-MSobsd 336.1960, calcd 336.1958[(M + H)$^+$, M = $C_{22}H_{25}NO_2$].

**15o:** 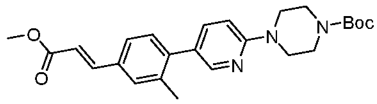

$^1$H NMR (300 MHz, $CDCl_3$)$\delta$821-8.20 (*d,* 1H), 7.74-7.69 (*d,* 1H), 7.53-7.41 (*m,* 3H), 7.25-7.23 (*d,* 1H), 6.74-6.71 (*d*, 1H), 6.50-6.45 (*d*, 1H), 3.83 (*s*, 3H), 3.60 (*s*, 8H),2.34 (*s*, 3H), 1.52 (*s*, 9H) ppm;ESI-MSobsd 438.2387, calcd 438.2387[(M + H)$^+$, M = $C_{25}H_{31}N_3O_4$].

**15p:** 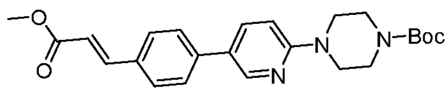

$^1$H NMR (300 MHz, $CDCl_3$)$\delta$8.52-8.51 (*d,* 1H), 7.75-7.70 (*m,* 2H), 7.61-7.55 (*m,* 3H), 7.30-7.28 (*m,* 1H), 6.75-6.72 (*d,* 1H), 6.52-6.46 (*d,* 1H), 3.85 (*s*, 3H), 3.62 (*s*, 8H),1.51 (*s*, 9H) ppm;ESI-MSobsd 424.2229, calcd 424.2231[(M + H)$^+$, M = $C_{24}H_{29}N_3O_4$].

**Embodiment 4**

Intermediate 15 (1 eq.) was dissolved in a mixed solvent of tetrahydrofuran and water (V/V = 1:1), lithium hydroxide (5 eq.) was added, stirred at room temperature for 2-3 hours, and the reaction was finished. The tetrahydrofuran was removed by rotary evaporation, the pH was adjusted to 2-3 with 1 N hydrochloric acid solution, then extracted with ethyl acetate for three times and combined with organic phases, washed with saturated brine, dried and concentrated. Compound 16 was obtained by column chromatography. 16 (1 eq.), O-(tetrahydro-2H-pyran-2-yl) hydroxylamine (2 eq.), EDCI (2 eq.), HOBt (2 eq.) was added into a round bottom flask, dissolved by addition of dichloromethane, and DIPEA (4 eq.) was added and stirred at room temperature for 1.5 hours. After the reaction was finished, water was used to quench the reaction, and then extracted with dichloromethane for three times, combined with the organic phases, washed with saturated brine, dried and concentrated. Compound 17a was obtained by column chromatography. In this way it was also possible to prepare 17b -17c and 17g -17p.

Intermediate 2c (1 eq.) and 2-methyl-2-butene (20 eq.) were dissolved in tert-butanol, and a mixed solution of sodium chlorite (10 eq.) and 20% sodium dihydrogen phosphate was added to the reaction system, stirred at room temperature overnight, and the reaction was finished. The reaction was quenched by the addition of saturated sodium sulfite, stirred for 15 min and monitored for pH to 7, then extracted with ethyl acetate for three times and combined with organic phases, washed with saturated brine, dried and concentrated. Compound 18 was obtained by column chromatography. Intermediate 18 (1 eq.), o-phenylenediamine 19 (1.2 eq.) and HOBt (2 eq.) were dissolved in dichloromethane and DIPEA (4 eq.) was added dropwise followed by EDCI (2 eq.) and stirred at room temperature overnight. After the reaction was finished, saturated ammonium chloride solution was used to quench the reaction, and then extracted with dichloromethane for three times, combined with the organic phases, washed with saturated brine, dried and concentrated. Compounds 17d-17f were obtained by column chromatography, and the reaction formula was as follows:

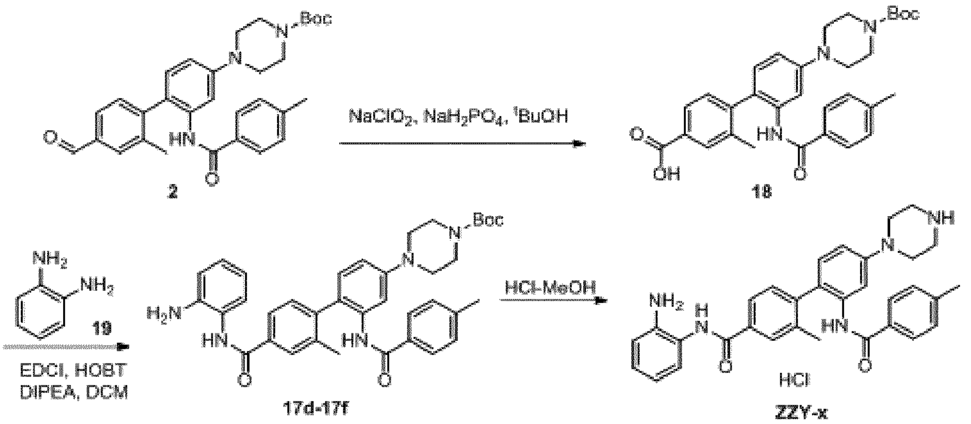


The structure and analytical data of 17a -17p are as follows:

17a: 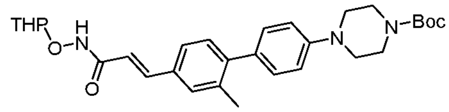

$^1$H NMR (500 MHz, $CDCl_3$)$\delta$8.32 (*sbr,* 1H), 7.76-7.72 (*d,* 1H), 7.40-7.37 (*d,* 2H), 7.25-7.23 (*m,* 3H), 6.98-6.96 (*d,* 2H),5.01 (*s*, 1H),4.00-3.96 (*m,* 1H), 3.68-3.66 (*d,* 1H),3.61-3.59 (*m,* 4H), 3.21-3.19 (*m,* 4H), 2.31 (*s*, 3H), 1.87-1.62 (*m,* 6H), 1.49 (*s*, 9H)ppm; ESI-MS obsd 522.2966, calcd 522.2962[(M + H)$^+$, M = $C_{30}H_{39}N_3O_5$].

17b: 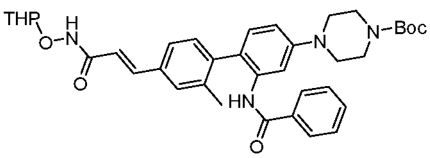

$^1$H NMR (400 MHz, $CDCl_3$)$\delta$8.34-8.33 (*d,* br, 1H), 7.83-7.71 (*m,* 2H), 7.64-7.62 (*m,* 1H), 7.57-7.47 (*m,* 3H), 7.41-7.36 (*m,* 1H),7.28-7.25 (*m,* 5H),7.11-7.08 (*d,* 1H), 6.80-6.77 (*m,* 1H),5.10-5.04 (*m,* 1H), 4.70-3.91 (*m,* 6H), 3.70-3.59 (*m,* 2H),3.35-3.25 (*m,* 2H), 2.18-2.07 (*s*, 3H), 1.80-1.62 (*m,* 2H), 1.55-1.52 (*s*, 9H), 1.50-1.32 (*m,* 4H)ppm; ESI-MS obsd 641.3334, calcd 641.3334[(M + H)$^+$, M = $C_{37}H_{44}N_4O_6$].

17c: 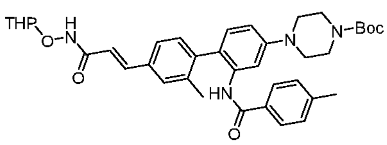

$^1$H NMR (400 MHz, $CDCl_3$)$\delta$8.32-8.31 (*d,* br, 1H), 7.80-7.76 (*d,* 1H), 7.56 (*s*, 1H), 7.49-7.46 (*m,* 2H), 7.36-7.34 (*m,* 2H),7.28 (*s*, 1H),7.17-7.15 (*m,* 2H),7.07-7.05 (*d,* 1H), 6.76-6.74 (*m,* 1H),5.04 (*s*, 1H),3.94-3.88 (*m,* 1H), 3.70-3.67 (*m,* 1H),3.62-3.60 (*m,* 4H), 3.29-3.26 (*m,* 4H), 2.35 (*s*, 3H), 2.15 (*s*, 3H), 1.94-1.72 (*m,* 6H), 1.50 (*s*, 9H)ppm; ESI-MS obsd 655.3497, calcd655.3490[(M + H)$^+$, M = $C_{38}H_{46}N_4O_6$].

17d: 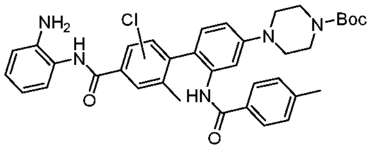

$^1$H NMR (500 MHz, $CDCl_3$)$\delta$8.29 (*sbr,* 1H), 7.92 (*s*, 2H), 7.83-7.82 (*d,* 1H), 7.52 (*s*, 1H), 7.40-7.38 (*m,* 4H),7.19-7.17 (*d,* 2H),7.14-7.11 (*m,* 1H),7.07-7.05 (*d,* 1H), 6.89-6.88 (*m,* 2H), 6.78-6.76 (*m,* 1H),5.30 (*sbr,* 1H),3.62-3.60 (*m,* 4H), 3.29-3.27 (*m,* 4H), 2.36 (*s*, 3H), 2.23 (*s*, 3H), 1.50 (*s*, 9H)ppm;ESI-MSobsd 654.2849, calcd 654.2842[(M + H)$^+$,

$M = C_{37}H_{40}ClN_5O_4$].

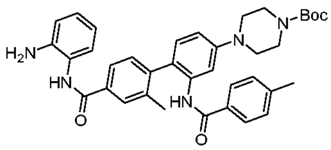

**17e:**

$^1H$ NMR (400 MHz, MeOH-$d_4$)$\delta$7.89-7.88 (*d,* 1H), 7.81-7.77 (*m,* 1H), 7.53-7.50 (*d,* 2H), 7.35-7.33 (*m,* 1H), 7.23-7.18 (*m,* 3H),7.10-7.01 (*m,* 2H), 6.91-6.89 (*m,* 1H), 6.79-6.75 (*m,* 1H), 6.72-6.68 (*m,* 2H), 6.61-6.58 (*m,* 2H),3.72-3.57 (*m,* 4H), 3.29-3.17 (*m,* 4H), 2.35 (*s*, 3H), 2.25 (*s*, 3H), 1.49 (*s*, 9H)ppm;ESI-MSobsd 620.3235, calcd 620.3231[$(M + H)^+$, $M = C_{37}H_{41}N_5O_4$].

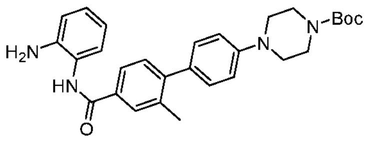

**17f:**

$^1H$ NMR (500 MHz, $CDCl_3$)$\delta$7.86 (*sbr,* 1H),7.83-7.81 (*m,* 1H), 7.75-7.72 (*m,* 1H), 7.37-7.32 (*m,* 2H),7.28-7.24 (*m,* 2H),7.13-7.08 (*m,* 1H),7.01-6.96 (*m,* 2H), 6.89-6.84 (*m,* 2H), 3.92 (*sbr,* 2H),3.64-3.58 (*m,* 4H), 3.25-3.17 (*m,* 4H), 2.37 (*s*, 3H), 1.50 (*s*, 9H)ppm;ESI-MSobsd 487.2707, calcd 487.2704[$(M + H)^+$, $M = C_{29}H_{34}N_4O_3$].

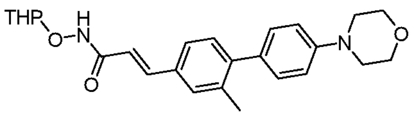

**17g:**

$^1H$ NMR (300 MHz, $CDCl_3$)$\delta$9.04 (*sbr,* 1H), 7.79-7.73 (*d,* 1H), 7.41-7.28 (*m,* 3H), 7.23 (*s*, 1H), 6.98-6.90 (*m,* 2H), 6.84-6.81 (*d,* 1H), 6.49 (*m,* 1H), 3.92-3.86 (*m,* 4H),3.70-3.60 (*m,* 2H), 3.23-3.20 (*m,* 4H), 2.31-2.28 (*d,* 3H), 1.88-1.56 (*m,* 6H)ppm;ESI-MSobsd 445.2103, calcd 445.2098[$(M + Na)^+$, $M = C_{25}H_{30}N_2O_4$].

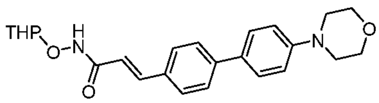

**17h:**

$^1H$ NMR (300 MHz, $CDCl_3$)$\delta$8.77 (*sbr,* 1H), 7.83-7.76 (*m,* 3H), 7.44-7.32 (*m,* 2H), 7.17-7.10 (*m,* 2H), 7.05-6.90 (*m,* 2H), 6.48 (*sbr,* 1H), 5.05 (*br,* 1H), 4.50-3.95 (*m,* 6H),3.90-3.60 (*m,* 4H), 1.88-1.56 (*m,* 6H) ppm;ESI-MSobsd 409.2127, calcd 409.2122[$(M + H)^+$, $M = C_{24}H_{28}N_2O_4$].

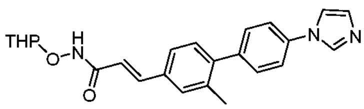

**17i:**

$^1H$ NMR (300 MHz, $CDCl_3$)$\delta$8.20 (*s*, 1H), 7.67-7.62 (*d,* 1H), 7.47-7.40 (*m,* 8H),7.28-7.26 (*d,* 2H), 7.17-7.15 (*d,* 1H), , 4.04 (*s*, 1H), 3.88-3.85 (*m,* 2H), 2.24 (*s*, 3H), 1.70-1.62 (*m,* 6H)ppm;ESI-MSobsd 404.1889, calcd 404.1969[$(M + H)^+$, $M = C_{24}H_{25}N_3O_3$].

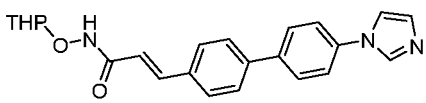

**17g:**

$^1H$ NMR (300 MHz, $CDCl_3$)$\delta$7.82-7.74 (*m,* 2H), 7.70-7.62 (*m,* 4H), 7.60-7.55 (*m,* 3H), 7.36-7.26 (*m,* 3H), 6.78-6.73 (*m,* 1H), 5.04-4.95 (*m,* 1H), 4.10-3.78 (*m,* 2H), 1.71-1.60 (*m,* 6H)ppm;ESI-MSobsd 390.1815, calcd 390.1812[$(M + H)^+$, $M = C_{23}H_{23}N_3O_3$].

**17k:** 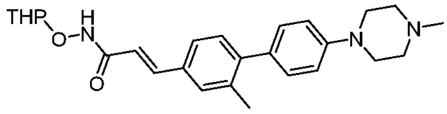

$^1$H NMR (300 MHz, $CDCl_3$)$\delta$7.87-7.84 (*d,* 1H), 7.75-7.63 (*d,* 2H), 7.35-7.32 (*d,* 1H), 7.15-7.13 (*d,* 2H), 6.87-6.78 (*m,* 2H), 6.56 (*s*, 1H), 5.08 (*s*, 1H),4.00 (*s*, 1H),3.62 (*s*, 1H), 3.37 (*m,* 4H), 3.05-3.01 (*m,* 4H), 2.66-2.62 (*m,* 3H), 2.22 (*s*, 3H), 1.85-1.59 (*m,* 6H) ppm;ESI-MSobsd 436.2599, calcd 436.2595[(M + H)$^+$, M = $C_{26}H_{33}N_3O_3$].

**17l:** 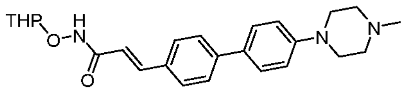

$^1$H NMR (300 MHz, $CDCl_3$)$\delta$7.86-7.83 (*d,* 1H), 7.76-7.71 (*d,* 1H), 7.65-7.63 (*d,* 1H), 7.50-7.46 (*d,* 4H),7.10-7.02 (*m,* 1H),6.87-6.84 (*d,* 1H), 6.59 (*s*, 1H), 5.09 (*s*, 1H),4.00 (*s*, 1H),3.61 (*s*, 1H), 3.40 (*s*, 4H), 3.14 (*s*, 4H), 2.72-2.70 (*s*, 3H), 1.85-1.60 (*m,* 6H) ppm;ESI-MSobsd 422.2438, calcd 422.2438[(M + H)$^+$, M = $C_{25}H_{31}N_3O_3$].

**17m:** 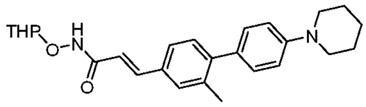

$^1$H NMR (300 MHz, $CDCl_3$) $\delta$8.61 (*s*br, 1H), 7.79-7.74 (*d,* 1H), 7.41-7.39 (*d,* 1H), 7.33-7.30 (*d,* 1H),7.26-7.21 (*m,* 2H), 7.01-6.93 (*m,* 2H), 6.87-6.76 (*m,* 1H), 5.04 (*s*, 1H),4.04-3.93 (*m,* 1H), 3.71-3.63 (*m,* 1H),3.26-3.21 (*m,* 4H), 2.33-2.31 (*s*, 3H), 1.89-1.85 (*m,* 2H), 1.79-1.70 (*m,* 6H), 1.63-1.56 (*m*, 4H) ppm; ESI-MS obsd 421.2424, calcd 421.2486[(M + H)$^+$, M = $C_{26}H_{32}N_2O_3$].

**17n:** 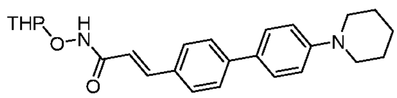

$^1$H NMR (300 MHz, $CDCl_3$) $\delta$8.82 (*s*br, 1H), 7.80-7.73 (*m,* 1H), 7.60-7.56 (*d,* 4H), 7.54-7.51 (*d,* 1H),7.36-7.33 (*m,* 1H), 7.07-6.95 (*m,* 2H), 6.54-6.46 (*m,* 1H), 5.05 (*s*, 1H),4.01-3.92 (*m,* 1H), 3.71-3.61 (*m,* 1H),3.23 (*s*, 4H), 1.89-1.85 (*m,* 2H), 1.75-1.74 (*m,* 6H), 1.63-1.61 (*m,* 4H) ppm; ESI-MS obsd 407.2325, calcd 407.2329[(M + H)$^+$, M = $C_{25}H_{30}N_2O_3$].

**17o:** 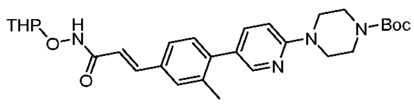

$^1$H NMR (300 MHz, $CDCl_3$) $\delta$9.30 (*sbr,* 1H), 8.17 (*d,* 1H), 7.77-7.71 (*d,* 1H), 7.50-7.47 (*m,* 1H), 7.40 (*s*, 2H), 7.20-7.17 (*d,* 1H), 6.72-6.69 (*d,* 1H), 6.49 (*s*, 1H), 5.05 (*s*, 1H),4.04-3.98 (*m,* 1H), 3.68-3.65 (*d,* 1H),3.53 (*s*, 8H), 2.34 (*s*, 3H), 1.87-1.62 (*m,* 6H), 1.50 (*s*, 9H) ppm; ESI-MS obsd 545.2720, calcd 545.2734[(M + Na)$^+$, M = $C_{29}H_{38}N_4O_5$].

**17p:** 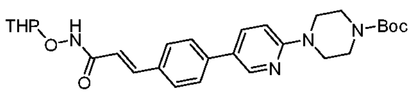

$^1$H NMR (300 MHz, $CDCl_3$) $\delta$8.49 (*d,* 2H), 7.81-7.78 (*d,* 1H), 7.75 (*s*, 1H), 7.61-7.54 (*m,* 4H), 6.75-6.72 (*d,* 1H),5.04 (*s*, 1H),4.04-3.98 (*m,* 1H), 3.71-3.67 (*d,* 1H),3.60 (*s*, 8H), 1.89-1.66 (*m,* 6H), 1.51 (*s*, 9H) ppm; ESI-MS obsd 531.2564, calcd 531.2578[(M +Na)$^+$, M = $C_{28}H_{36}N_4O_5$].

**Embodiment 5**

Intermediate 17 (e.g. 17a - 17p) was dissolved in methanol and then added dropwise to a 2 N hydrogen chloride

in methanol (2 eq.), stirred at room temperature for 30 min, most of the methanol was removed by rotary evaporation after the reaction was monitored by TLC, the system was kept clear, ether was added to precipitate a solid, standing was carried out, the solvent was sucked out, and dried to obtain the product ZZY-001 to ZZY-016 series molecules. The above operation can also be done with trifluoroacetic acid and dichloromethane system, and the final product molecules obtained have different carboxylic acid pairing ions.

For example, the reaction formula for the generation of ZZY-001 was as follows:

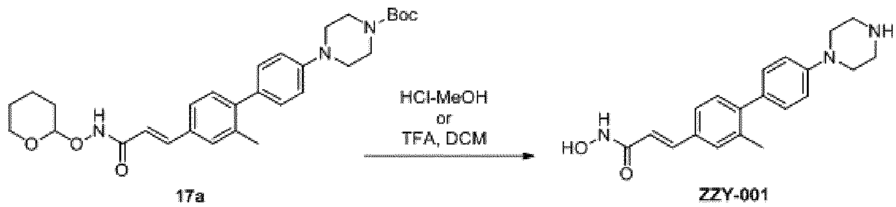


17a ZZY-001

The structure and analytical data of ZZY-001 to ZZY-016 are as follows:

**ZZY-001:**

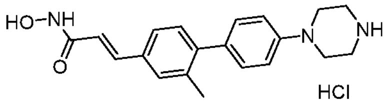

$^1$H NMR (500 MHz, MeOH-*d4*) $\delta$7.90 (sbr, 1H), 7.61-7.58 (*d,* 1H), 7.46-7.42 (*m,* 2H), 7.29-7.27 (*d,* 2H), 7.22-7.20 (*d,* 1H), 7.13-7.12 (*m,* 2H), 6.50-6.47 (*d,* 1H), 3.50-3.49 (*m,* 4H), 3.43-3.42 (*m,* 4H), 2.28 (*s*, 3H) ppm; ESI-MS obsd 338.1862, calcd 338.1863[(M + H)$^+$, M = $C_{20}H_{23}N_3O_2$].

**ZZY-002:**

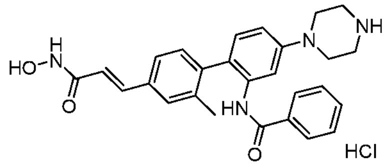

$^1$H NMR (500 MHz, MeOH-*d4*) $\delta$7.58-7.54 (*m,* 3H), 7.52-7.48 (*m,* 3H), 7.44-7.37 (*m,* 3H), 7.24-7.20 (*m,* 2H), 7.06-7.03 (*m,* 1H), 6.49-6.45 (*d,* 1H), 3.54-3.51 (*m,* 4H), 3.43-3.40 (*m,* 4H), 2.17 (*s*, 3H) ppm; ESI-MS obsd 457.2235, calcd 457.2234[(M + H)$^+$, M = $C_{27}H_{28}N_4O_3$].

**ZZY-003:**

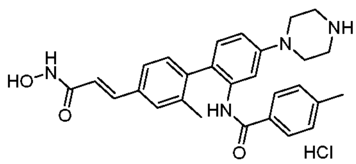

$^1$H NMR (500 MHz, MeOH-*d4*) $\delta$7.74(*s*, 1H), 7.64-7.60(*d*, 1H), 7.48-7.42(*m*, 4H), 7.32-7.26 (*m,* 2H), 7.23-7.15 (*m,* 3H), 6.57-6.54 (*m,* 1H), 3.77 (*s*, 4H), 3.60 (*s*, 4H), 2.31 (*s*, 3H), 2.14 (*s*, 3H) ppm; ESI-MS obsd 471.2390, calcd 471.2391[(M + H)$^+$, M = $C_{28}H_{30}N_4O_3$].

**ZZY-004:**

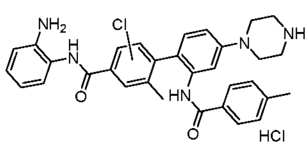

$^1$H NMR (400 MHz, MeOH-*d4*) $\delta$ 7.61-7.18 (*m,* 15H), 3.48-3.37 (*m,* 8H), 2.36 (*s*, 3H), 2.28 (*s*, 3H) ppm; ESI-MS obsd 554.2235, calcd 554.2317[(M + H)$^+$, M = $C_{32}H_{32}ClN_5O_2$].

ZZY-005: 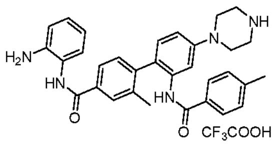

$^1$H NMR (500 MHz, MeOH-*d4*) $\delta$7.94 (*sbr,* 1H), 7.86-7.85 (*d,* 1H), 7.53-7.51 (*d,* 2H), 7.38-7.33 (*m,* 4H), 7.30-7.27 (*m,* 2H),7.23-7.20 (*m*, 2H),7.09-7.07 (*m,* 1H),5.49 (*s*br, 1H),3.54-3.52 (*m,* 4H), 3.43-3.40 (*m*, 4H), 2.35 (*s*, 3H), 2.26 (*s*, 3H) ppm; ESI-MS obsd 520.2708, calcd 520.2707[(M + H)$^+$, M = $C_{32}H_{33}N_5O_2$].

ZZY-006: 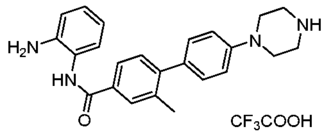

$^1$H NMR (400 MHz, MeOH-*d4*) $\delta$7.95 (sbr, 1H), 7.89-7.86 (*d,* 1H), 7.38-7.29 (*m,* 5H), 7.26-7.20 (*m,* 2H),7.14-7.12 (*m,* 2H),3.49-3.48 (*m,* 4H), 3.42-3.39 (*m,* 4H), 2.36 (*s*, 3H) ppm; ESI-MS obsd 387.2183, calcd 387.2179 [(M + H)$^+$, M = $C_{24}H_{26}N_4O$].

ZZY-007: 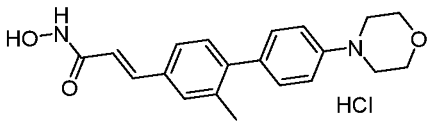

$^1$H NMR (300 MHz, MeOH-*d4*) $\delta$7.91-7.82(*m,* 2H), 7.61-7.48 (*m,* 5H), 7.32-7.24 (*m,* 1H), 6.59-6.54 (*d,* 1H), 4.20 (*s*, 4H), 3.82 (*s*, 4H), 2.32-2.29 (*d,* 3H) ppm; ESI-MS obsd 339.1715, calcd 339.1703 [(M + H)$^+$, M = $C_{20}H_{22}N_2O_3$].

ZZY-008: 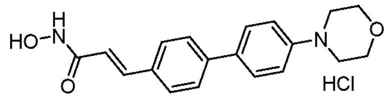

$^1$H NMR (300 MHz, MeOH-*d4*) $\delta$8.07 (*sbr,* 1H), 7.91-7.88 (*m,* 2H),7.82-7.79 (*d,* 1H), 7.76-7.74 (*d,* 2H), 7.71-7.62 (*m,* 4H), 6.60-6.55 (*d*, 1H), 4.17-4.16 (*d*, 4H), 3.82-3.76 (*m,* 4H) ppm; ESI-MS obsd 325.1549, calcd 325.1547[(M + H)$^+$, M = $C_{19}H_{20}N_2O_3$].

ZZY-009: 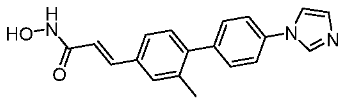

$^1$H NMR (300 MHz, MeOH-*d4*) $\delta$9.59 (*sbr,* 1H), 8.18 (*s*, 1H), 7.87-7.84 (*d,* 3H),7.65-7.50 (*m,* 5H), 7.32-7.29 (*d,* 1H), 6.59-6.54 (*d,* 1H), 2.33 (*s*, 3H) ppm; ESI-MS obsd 320.1390, calcd 320.1394 [(M + H)$^+$, M = $C_{19}H_{17}N_3O_2$].

ZZY-010: 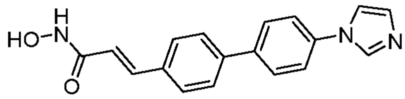

$^1$H NMR (400 MHz, MeOH-*d4*) $\delta$9.54 (*sbr,* 1H), 8.15 (*s*, 1H), 7.95-7.91 (*m,* 3H),7.85-7.81 (*m,* 4H),7.76-7.74 (*d,* 3H),7.70-7.68 (*m,* 3H),7.64-7.60 (*d,* 1H), 6.59-6.55 (*d,* 1H)ppm; ESI-MS obsd 306.1231, calcd 306.1237[(M + H)$^+$, M = $C_{18}H_{15}N_3O_2$].

ZZY-011: 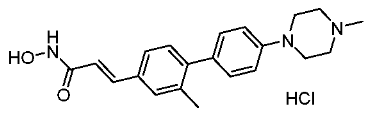

$^1$H NMR (300 MHz, MeOH-*d4*) $\delta$7.65-7.60(*d*, 1H), 7.48-7.42 (*m,* 2H), 7.29-7.11 (*m,* 2H),7.16-7.13 (*m,* 2H), 7.02-6.92 (*m,* 1H), 6.57-6.52 (*d,* 1H), 3.96-3.64 (*m,* 4H), 3.33-3.19 (*m,* 4H), 3.01-3.00 (*s*, 3H), 2.28 (*s*, 3H) ppm; ESI-MS obsd 352.2020, calcd 352.2020[(M + H)$^+$, M = $C_{21}H_{25}N_3O_2$].

ZZY-012: 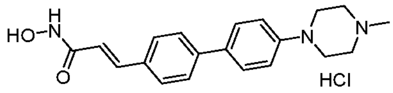

$^1$H NMR (300 MHz, MeOH-*d4*) $\delta$7.70-7.59 (*m,* 6H), 7.43-7.38 (*m,* 1H), 7.30-7.23 (*m,* 1H),7.14-7.06 (*m,* 1H), 6.57-6.49 (*m,* 1H), 3.97-3.91 (*m,* 2H), 3.68-3.61 (*m,* 2H), 3.34-3.29 (*m*, 2H), 3.21-3.13 (*m,* 2H), 3.01-3.00 (*s*, 3H) ppm; ESI-MS obsd 338.1865, calcd 338.1863[(M + H)$^+$, M = $C_{20}H_{23}N_3O_2$].

ZZY-013: 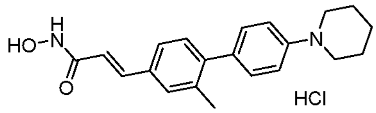

$^1$H NMR (300 MHz, MeOH-*d4*) $\delta$7.83-7.80 (*d,* 1H), 7.74-7.71 (*m,* 2H), 7.61-7.50 (*m,* 5H),7.32-7.25 (*m,* 1H), 6.74-6.55 (*m,* 1H), 3.75-3.72 (*m,* 4H), 2.33-2.30 (*s*, 3H), 2.13-2.11 (*m,* 4H),1.87 (*s*, 2H) ppm; ESI-MS obsd 337.1916, calcd 337.1911[(M + H)$^+$, M = $C_{21}H_{24}N_2O_2$].

ZZY-014: 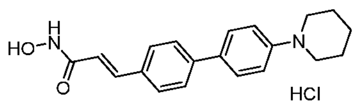

$^1$H NMR (300 MHz, MeOH-*d4*) $\delta$7.88-7.71 (*m,* 9H), 6.73-6.59 (*m,* 1H), 3.71 (*sbr,* 4H), 2.12 (*sbr,* 4H); 1.86 (br*s*, 2H) ppm; ESI-MS obsd 323.1761, calcd 323.1754[(M + H)$^+$, M = $C_{20}H_{22}N_2O_2$].

ZZY-015: 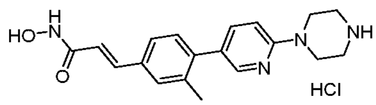

$^1$H NMR (300 MHz, MeOH-*d4*) $\delta$8.21-8.18 (*d,* 1H), 8.07 (*s*, 1H), 7.60-7.50 (*d,* 4H), 7.33-7.31 (*d,* 1H), 6.59-6.54 (*d,* 1H), 4.15 (*sbr,* 4H),3.81 (*sbr,* 4H), 2.04 (*s*, 3H) ppm;ESI-MS obsd 339.1812, calcd 339.1816[(M + H)$^+$, M = $C_{19}H_{22}N_4O_2$].

ZZY-016: 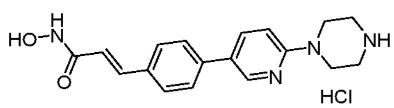

$^1$H NMR (300 MHz, MeOH-*d4*) $\delta$8.50-8.46 (*m,* 1H), 8.38-8.37 (*d,* 1H), 7.77-7.69 (*m,* 4H), 7.63-7.59 (*d,* 1H), 7.58-7.56 (*d,* 1H), 6.59-6.54 (*d,* 1H), 4.11-4.07 (*m,* 4H),3.55-3.51 (*m,* 4H) ppm;ESI-MS obsd 325.1656, calcd 325.1659[(M + H)$^+$, M = $C_{18}H_{20}N_4O_2$].

**Embodiment 6 In vitro LSD1 inhibitory activity detection**

LSD1 activity was detected by using LSD1-HRP coupling reaction. The principle was shown in the following reaction formula: as can be seen from the reaction mechanism of substrate demethylation catalyzed by LSD1, by-product $H_2O_2$ was produced in this process, thus horseradish peroxidase (HRP) was used to catalyze the reaction of $H_2O_2$ with

Amplex Red (a dye) to produce Resorufin (a highly fluorescent substance) and $H_2O$, and indirectly obtaining the inhibitory activity of the molecule to be tested on the LSD1 by detecting the fluorescence intensity of the product. During the reduction of $H_2O_2$ to $H_2O$, Amplex Red acts as an electron donor and oxidizes to Resorufin for detection of fluorescence. In this experiment, 535 nm was selected as the excitation wavelength and 595 nm as the emission wavelength for detection. Ampliflu™ Red, peroxidase, from horseradish required for the experiment was purchased from SIGMA; H3K4Me2 substrate peptide was purchased from GL Biochemical (Shanghai) Co., Ltd.

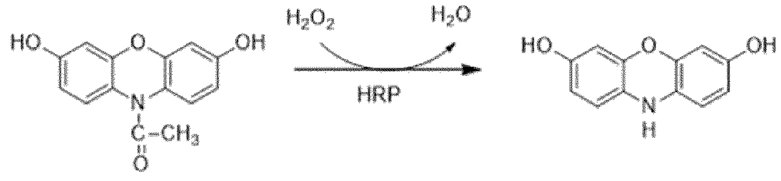


Amplex Red　　　Resorufin

During the test, the recombinant human LSD1 protein with enzyme activity was first expressed in E. coli, and then the samples were prepared according to the 50 μl/well system: containing 10 μM HRP + 50 μM Amplex Red + 100 nM LSD1+buffer (25 mM Hepes, 250 mM NaCl, 5% Glycerol pH 7.5), added to the 96-well plate; the gradient diluted molecules to be tested were added to the 96-well plate, and the oscillatory reaction was carried out for 30 min at room temperature; the starting solution was prepared according to 50 μl/well system: containing 10 μM H3K4Me2 peptide + buffer, added to the 96-well plate, and after slight shaking, immediately detected by multi-function microplate enzyme marker (Ex: 535 nM; Em: 595 nM). The results are shown in Table 1 below: Table 1 shows the inhibitory activity of the compound of the present invention on blocking LSD1 enzyme activity in vitro.

Table 1

| Compoun ds | $IC_{50}$ (nM) | Compounds | $IC_{50}$ (nM) | Compound s | $IC_{50}$ (nM) | Compound s | $IC_{50}$ (nM) |
|---|---|---|---|---|---|---|---|
| **ZZY-001** | 1549 | **ZZY-005** | 5164 | **ZZY-009** | 1405 | **ZZY-013** | 1415 |
| **ZZY-002** | 1320 | **ZZY-006** | 2376 | **ZZY-010** | 8264 | **ZZY-014** | 1106 |
| **ZZY-003** | 439.9 | **ZZY-007** | 477 | **ZZY-011** | 131.3 | **ZZY-015** | 879.2 |
| **ZZY-004** | 2487 | **ZZY-008** | 715.6 | **ZZY-012** | 16.09 | **ZZY-016** | 991.1 |
| **TCP** | >30,000 | **Vorinostat** | >100,000 | | | | |

It can be seen from the $IC_{50}$ (nM) values of Table 1 that the ability of the compounds ZZY-001 to ZZY-016 to inhibit LSD1 was far superior to the LSD1 positive inhibitor TCP (tranylcypromine) and the HDAC positive inhibitor Vorinostat, so compounds ZZY-001 to ZZY-016 are highly effective inhibitors of LSD1.

**Embodiment 7 In vitro HDAC1 inhibitory activity detection**

HDAC1 inhibitory activity was detected by using the FLUOR DE LYS® HDAC1 fluorometric drug discovery assay kit. The principle was shown in the following reaction formula: HDAC1 catalyzes the deacetylation of a substrate FLUOR DE LYS® Substrate (containing an acetylated side chain), the product can react with FLUOR DE LYS® Developer II to generate fluorescence (shown in the following reaction process), and the inhibitory activity of a molecule to be detected on HDAC1 can be indirectly obtained by detecting the fluorescence intensity of the product. In this experiment, 360 nm was selected as the excitation wavelength and 460 nm as the emission wavelength for detection. FLUOR DE LYS® HDAC1 fluorometric drug discovery assay kit required for experiments was purchased from Enzo Biochem Inc.

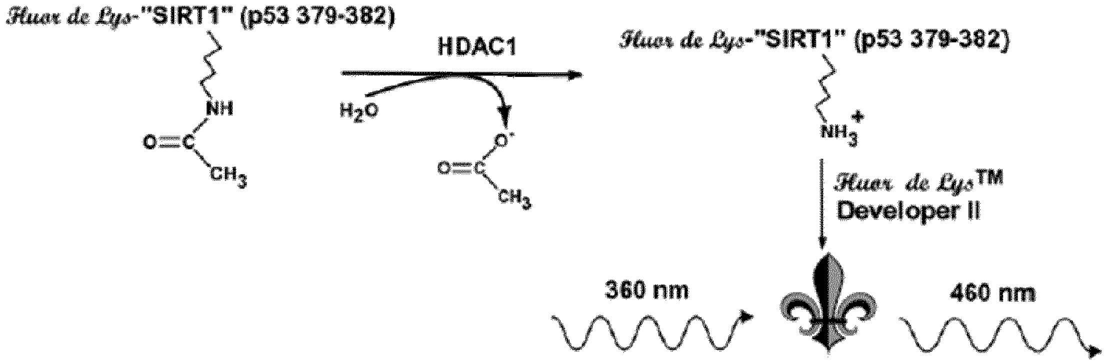


During the test, the sample was prepared according to the specific experimental conditions of blocking the HDAC1 enzyme activity of the compound of the present invention in vitro in accordance with Table 2 below. After slight oscillation at the end of the third step, the samples were detected immediately by multifunctional microplate enzyme marker (Ex: 360 nM; Em: 460 nM). The results are shown in Table 3 below: Table 3 shows the inhibitory activity of compounds of the present invention to block HDAC1 enzyme activity in vitro.

Table 2

| | **The first step (reacting for 30 min at 30°C after oscillation)** | | | **The second step (reacting for 60 min at 30°C after oscillation)** | **The third step (reacting for 45 min at 30°C after oscillation)** |
|---|---|---|---|---|---|
| **Sample** | HDAC Assay Buffer II | HDAC1 (Dilution) | Inhibitor (5x) | Fluor de Lys®-SIRT1 Substrate (BML-KI177) (2x) | Fluor de Lys® Developer II |
| **Test Sample** | 0 ul | 15 ul | 10 ul | 25 ul | 50 ul |
| **Positive control** | 10 ul | 15 ul | 0 ul | 25 ul | 50 ul |
| **Negative control** | 25 ul | 0 ul | 0 ul | 25 ul | 50 ul |

Table 3

| **Compounds** | **$IC_{50}$ (nM)** | **Compounds** | **$IC_{50}$ (nM)** | **Compounds** | **$IC_{50}$ (nM)** | **Compounds** | **$IC_{50}$ (nM)** |
|---|---|---|---|---|---|---|---|
| **ZZY-001** | 670.8 | **ZZY-005** | 8943 | **ZZY-009** | 2801 | **ZZY-013** | 9643 |
| **ZZY-002** | 71.57 | **ZZY-006** | 5385 | **ZZY-010** | 3522 | **ZZY-014** | 9855 |
| **ZZY-003** | 179.9 | **ZZY-007** | 1112 | **ZZY-011** | 434 | **ZZY-015** | 262.4 |
| **ZZY-004** | 12365 | **ZZY-008** | 9358 | **ZZY-012** | 942.4 | **ZZY-016** | 451.2 |
| **TCP** | >100,000 | **Vorinostat** | 206.0 | | | | |

It can be seen from the $IC_{50}$ (nM) values of Table 3 that the ability of the compounds ZZY-001 to ZZY-016 to inhibit HDAC1 was similar to HDAC positive inhibitor vorinostat, and was far superior to the LSD1 positive inhibitor TCP, so compounds ZZY-001 to ZZY-016 are highly effective inhibitors of HDAC1.

It can be seen by combining the $IC_{50}$ (nM) values of Tables 1 and Table 3: compounds of the embodiments ZZY-001 to ZZY-016 can simultaneously inhibit the activity of LSD1 and HDAC1, and are bifunctional inhibitors of LSD1 and HDAC.

**Embodiment 8 In vitro tumor cell inhibitory activity detection of the compound of the present invention**

MTT colorimetric method was used to detect the inhibitory activity of a series of compounds (i.e. ZZY-001 to

ZZY-016, a total of 16 kinds) of the embodiment of the present invention on the proliferation of tumor cells in vitro. The full name of MTT is 3- (4,5-dimethyl-2-thiazolyl)-2,5-diphenyl-2-H-tetrazolium romide, which is a yellow dye also known as thiazolyl blue. The detection principle was as follows: succinate dehydrogenase in the mitochondria of living cells reduced MTT to water-insoluble blue-violet crystalline Formazan, but dead cells do not have this function. DMSO can dissolve Formazan deposited in cells and the absorbance value can be measured with a enzyme marker at a wavelength of 490 nm. In a certain range of cell number, the production of Formazan is proportional to the number of cells, so the number of living cells can be inferred according to the measured OD value.

In the experiments of this embodiment, the selected cell lines are human breast cancer cell line MDA-MB-231 and BT-474, human colon adenocarcinoma cell HCT116, mouse colon cancer cell CT26.WT, mouse breast cancer cell 4T1. During the test, the cells in logarithmic phase and in good condition were digested with 0.25% trypsin digestion solution to make the adherent cells fall off, and the cell suspension (suspension cells without trypsin digestion) was made by counting 2-4$\times 10^4$ cells/ml. The cell suspension was inoculated on a 96-well plate at 100 $\mu$l/well and cultured in a constant temperature $CO_2$ incubator for 24 h. Then the tested drugs were added, 100 $\mu$l/well, and cultured for 72 h. MTT was added to a 96-well plate, 20 $\mu$l/well, and reacted for 4 h in the incubator. Absorb the supernatant, add DMSO, 150 $\mu$l/well and shake for 10 min on the plate shaker. The optical density (OD value) of each well was measured by enzyme marker at the wavelength of 490 nm. The results are shown in Table 4 below: Table 4 shows that compounds of several embodiments, ZZY-001, ZZY-002, ZZY-003, ZZY-011 and ZZY-012 have significant inhibitory activities on several human tumor cells HCT116, MDA-MB-231, BT-474, and mouse tumor cells CT26.WT and 4T1, wherein, the positive inhibitor of LSD1, TCP (anti-amphetamine), and the positive inhibitor of HDAC, Vorinostat, served as control.

Table 4

| **Compounds** | **$IC_{50}$ (nM)** | | | | |
|---|---|---|---|---|---|
| | **HCT116** | **MDA-MB-231** | **BT-474** | **CT26.WT** | **4T1** |
| **ZZY001** | 1540 | 2316 | 17826 | 2205 | 3869 |
| **ZZY002** | 1288 | 1368 | 7564 | 31153 | 5607 |
| **ZZY003** | 265.4 | 835.6 | 1861 | 8759 | 2379 |
| **ZZY011** | 728.2 | 2037 | 5904 | 1047 | 2597 |
| **ZZY012** | 468.7 | 1139 | 4214 | 736.7 | 2317 |
| **TCP** | >10,000 | >10,000 | >10,000 | >10,000 | >10,000 |
| **Vorinostat** | 1445 | 1580 | 3658 | 4355 | 2693 |

**Embodiment 9 Cell proliferation of 50 cell lines by the inventive compound ZZY-003**

The effect of the present invention compound ZZY-003 on the cell proliferation of 50 cell lines was evaluated by CellTiter-Glo (CTG) method, and the 50% inhibitory concentration was calculated by detecting the cell viability after treatment with different drug concentrations.

The cells were resuscitated and cultured in their respective culture media. The cells in logarithmic growth phase were harvested and the cells were counted by cell counter. The cell viability was detected by Trypan blue exclusion method to ensure that the cell line viability was more than 96%. The cell concentration was adjusted by diluting with the culture medium, and 90 $\mu$L cell suspension was added to the 96-well cell plate (including the cell control T0 on the day of drug treatment) to make the cell density reach the specified concentration. The cells in the 96-well plate were cultured overnight at 37°C, 5% $CO_2$ and 95% humidity. 10 $\mu$L culture medium was added to the control cell culture plate. CellTiter-Glo reagent and cell culture plate were placed at room temperature to balance for 30 minutes. The same volume of CellTiter-Glo reagent was added to each well. Vibrate on the orbital shaker for 2 min to make the cells fully lytic. The cell culture plate was placed at room temperature to balance for 10 minutes. Chemiluminescence values were read with EnVision. The storage solution was formed by dissolving the tested compound with the corresponding solvent and diluted in gradient to obtain 10 times working concentration solution, and 10 times solution of positive drug was also prepared. 10 $\mu$L drug solution was added to each well in the 96-well plate in which the cells were inoculated, and each cell concentration was provided with three duplicate wells. The highest concentration of the tested compound was 10/50 $\mu$M, 9 concentrations and 3.16 times dilution. The cells in the 96-well plate added with drug were cultured at 37°C, 5% $CO_2$ and 95% humidity for 72 hours. CellTiter-Glo reagent and drug-treated cell culture plate were placed at room temperature to balance for 30 minutes. The same volume of CellTiter-Glo reagent was added to each well. Vibrate on the orbital shaker for 2 minutes to make the cells fully lytic. The cell culture plate was placed at room temperature to

balance for 10 minutes. Chemiluminescence values were read with EnVision.

Data processing: GraphPad Prism 5.0 software was used to analyze the data, nonlinear S-curve regression was used to fit the data to get the dose-effect curve, and the $IC_{50}$ value was calculated. The specific data are shown in Table 5, and Table 5 shows the $IC_{50}$ value and the highest concentration inhibition rate of compound ZZY-003 on 50 cell lines.

Cell survival rate (%) = (Lum drug to be detected - Lum culture medium control)/(Lum cell control - Lum culture medium control) × 100%;

Lum cell control - Lum culture medium control was set to 100%, LumMedium control value was set to 0%;

amplification fold = (5th day LumNone treated - LumMedium control)/(2nd day LumNone treated - LumMedium control).

Table 5

| Cell No. | Cell lines | Absolute IC50(μM) | | % inhibition at top conc. | |
|---|---|---|---|---|---|
| | | ZZY-003 | Cisplatin | ZZY-003 | Cisplatin |
| 1 | **LP-1** | 0.26 | 8.68 | 98.94% | 99.89% |
| 2 | **MDA-MB-231** | 1.81 | 20.61 | 92.06% | 85.71% |
| 3 | **NCI-H1299** | 3.40 | 3.69 | 99.94% | 98.91% |
| 4 | **AN3 CA** | 1.27 | 4.43 | 99.92% | 99.96% |
| 5 | **SK-MEL-28** | 1.24 | 8.61 | 99.89% | 99.34% |
| 6 | **SNU-761** | 9.32 | 4.52 | 99.35% | 99.92% |
| 7 | **MDA-MB-453** | 0.19 | 12.92 | 99.93% | 95.15% |
| 8 | **143B** | 2.25 | 1.15 | 99.97% | 99.94% |
| 9 | **PC-3** | 1.38 | 5.34 | 96.56% | 85.52% |
| 10 | **MCF7** | 1.61 | 21.96 | 96.26% | 90.30% |
| 11 | **SNU-5** | 1.64 | 2.45 | 99.83% | 97.87% |
| 12 | **A549** | 2.21 | 7.89 | 95.22% | 96.72% |
| 13 | **PANC-1** | 5.84 | 8.67 | 96.79% | 94.45% |
| 14 | **SW480** | 1.74 | 2.69 | 99.24% | 94.12% |
| 15 | **EBC-1** | 1.15 | 17.77 | 92.30% | 85.11% |
| 16 | **5637** | 0.82 | 2.60 | 99.95% | 99.05% |
| 17 | **MOLM-13** | 0.21 | 0.77 | 99.99% | 99.99% |
| 18 | **NCI-H82** | 0.37 | 4.00 | 99.94% | 99.63% |
| 19 | **SK-OV-3** | 1.59 | 5.17 | 99.68% | 89.16% |
| 20 | **SK-N-FI** | 3.17 | 10.18 | 99.95% | 99.81% |
| 21 | **B16-F10** | 1.03 | 4.53 | 99.98% | 99.97% |
| 22 | **MIA PaCa-2** | 0.48 | 6.05 | 99.94% | 99.18% |
| 23 | **SU-DHL-6** | 0.30 | 5.65 | 99.91% | 99.95% |
| 24 | **U266B1** | 0.64 | 14.31 | 98.03% | 98.85% |
| 25 | **H69AR** | 4.36 | 5.65 | 96.23% | 94.93% |

(continued)

| Cell No. | Cell lines | Absolute IC50(μM) | | % inhibition at top conc. | |
|---|---|---|---|---|---|
| | | ZZY-003 | Cisplatin | ZZY-003 | Cisplatin |
| 26 | **DLD-1** | 5.13 | 5.30 | 97.98% | 98.93% |
| 27 | **CNE-2Z** | 1.52 | 1.18 | 99.84% | 96.44% |
| 28 | **HeLa** | 1.40 | 0.37 | 98.88% | 99.45% |
| 29 | **K-562** | 0.46 | 6.07 | 99.85% | 97.45% |
| 30 | **Jurkat clone E6-1** | 0.48 | 0.84 | 99.61% | 99.96% |
| 31 | **U-937** | 0.46 | 2.19 | 99.99% | 99.97% |
| 32 | **HL-60** | 0.31 | 1.56 | 99.93% | 99.99% |
| 33 | **HuCCT1** | 1.35 | 11.28 | 99.88% | 89.54% |
| 34 | **JVM-3** | 0.35 | 0.70 | 99.48% | 99.71% |
| 35 | **Ramos** | 0.32 | 1.78 | 99.82% | 99.98% |
| 36 | **OE19** | 0.78 | 25.81 | 98.95% | 62.99% |
| 37 | **BT474** | 0.51 | 66.09 | 99.86% | 67.02% |
| 38 | **Hep G2** | 0.84 | 5.86 | 99.98% | 90.70% |
| 39 | **T84** | 9.95 | 5.05 | 98.70% | 87.03% |
| 40 | **AMO-1** | 1.52 | 0.95 | 99.95% | 99.99% |
| 41 | **HCT-116** | 0.43 | 3.15 | 99.94% | 95.72% |
| 42 | **AZ-521** | 0.68 | 2.89 | 99.95% | 99.92% |
| 43 | **NCI-H1417** | 0.33 | 4.40 | 97.14% | 81.11% |
| 44 | **SF268** | 1.09 | 2.87 | 99.82% | 97.98% |
| 45 | **UO.31** | 4.56 | 4.23 | 99.92% | 97.89% |
| 46 | **786-O** | 3.91 | 2.11 | 99.96% | 97.88% |
| 47 | **LN-229** | 1.57 | 11.82 | 99.97% | 97.32% |
| 48 | **HT-1376** | 2.28 | 4.10 | 99.41% | 88.74% |
| 49 | **OPM2** | 0.35 | 3.67 | 98.99% | 99.97% |
| 50 | **MCF 10A** | 1.49 | 13.20 | 97.73% | 99.51% |

The data from Table 5 shows: the compound ZZY-003 of the present invention has significant inhibitory activity on 50 kinds of tumor cells, and the inhibitory activity on most of the detected tumor cells is stronger than that of the control chemotherapy drug Cisplatin, which fully shows that the compound of the present invention has broad-spectrum anti-tumor activity.

While the foregoing is directed to the preferred examples of the present invention, it is not intended to limit the present invention to the precise form disclosed, any modification, equivalent substitution and improvement made within the spirit and principle of the present application should be included in the protection scope of the present application.

## Claims

1. A compound, having a general structural formula as shown in Formula I:

X-AB-Y Formula I

In the above Formula I,

X is selected from any one of $-CO_2H$, -CONHZ, $-CH=CH-CO_2H$, -CH=CH-CONHZ, wherein Z is selected from any one of substituted or unsubstituted $C_1$-$C_{12}$ alkyl, substituted or unsubstituted aryl, and hydroxyl;
Y = $-NR_1R_2$, wherein $NR_1R_2$ is a substituted or unsubstituted 3- to 9-membered nitrogen-containing heterocycloalkyl;
A and B are each independently selected from substituted or unsubstituted phenylene, substituted or unsubstituted azaphenylene.

**2.** The compound of claim 1, wherein in the Formula I, A and B are each independently selected from: any one of

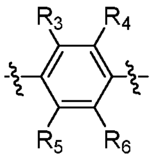 、 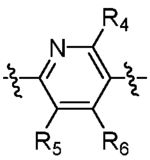 、 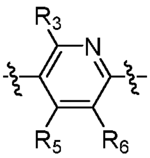 、 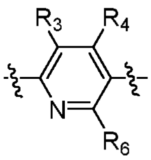 、 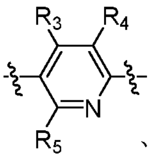 、 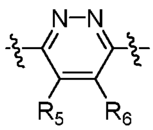 、 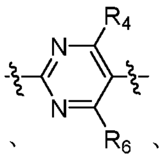 、

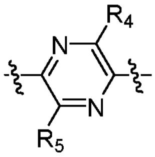 、 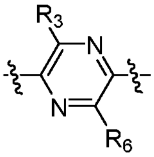 、 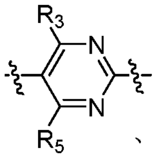 、 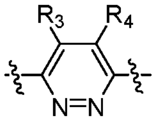 ;

Wherein $R_3$, $R_4$, $R_5$ and $R_6$ are each independently selected from any one of substituted or unsubstituted $C_1$-$C_{12}$ alkyl, substituted or unsubstituted $C_1$-$C_{12}$ heteroalkyl, substituted or unsubstituted $C_3$-$C_{12}$ cycloalkyl, substituted or unsubstituted $C_3$-$C_{12}$ heterocycloalkyl, substituted or unsubstituted $C_2$-$C_{12}$ alkenyl, substituted or unsubstituted $C_2$-$C_{12}$ heteroalkenyl, substituted or unsubstituted $C_3$-$C_{12}$ cycloalkenyl, substituted or unsubstituted $C_3$-$C_{12}$ heterocycloalkenyl, substituted or unsubstituted $C_2$-$C_{12}$ alkynyl, substituted or unsubstituted $C_2$-$C_{12}$ heteroalkynyl, substituted or unsubstituted $C_3$-$C_{12}$ cycloalkynyl, substituted or unsubstituted $C_3$-$C_{12}$ heterocycloalkynyl, substituted or unsubstituted aryl, substituted or unsubstituted heteroaryl, aryl ($C_1$-$C_{12}$) alkyl, heteroaryl ($C_1$-$C_{12}$) alkyl, $C_2$-$C_{12}$ alkenyl ($C_1$-$C_{12}$) alkyl, $C_2$-$C_{12}$ alkynyl ($C_1$-$C_{12}$) alkyl, hydroxyl, amino, nitro, sulfo, halogen and hydrogen atom.

**3.** The compound of claim 2, wherein in the Formula I, $R_3$, $R_4$, $R_5$ and $R_6$ are each independently selected from any one of substituted or unsubstituted $C_1$-$C_6$ alkyl, substituted or unsubstituted $C_1$-$C_6$ heteroalkyl, substituted or unsubstituted $C_3$-$C_6$ cycloalkyl, substituted or unsubstituted $C_3$-$C_6$ heterocycloalkyl, substituted or unsubstituted $C_2$-$C_6$ alkenyl, substituted or unsubstituted $C_2$-$C_6$ heteroalkenyl, substituted or unsubstituted $C_3$-$C_6$ cycloalkenyl, substituted or unsubstituted $C_3$-$C_6$ heterocycloalkenyl, substituted or unsubstituted $C_2$-$C_6$ alkynyl, substituted or unsubstituted $C_2$-$C_6$ heteroalkynyl, substituted or unsubstituted $C_3$-$C_6$ cycloalkynyl, substituted or unsubstituted $C_3$-$C_6$ heterocycloalkynyl, substituted or unsubstituted aryl, substituted or unsubstituted heteroaryl, aryl ($C_1$-$C_6$) alkyl, heteroaryl ($C_1$-$C_6$) alkyl, $C_2$-$C_6$ alkenyl ($C_1$-$C_6$) alkyl, $C_2$-$C_6$ alkynyl ($C_1$-$C_6$) alkyl.

**4.** The compound of claim 2, wherein in the Formula I, a structure of -AB- is selected from: any one of

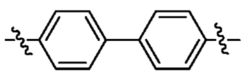 、 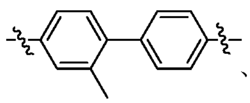 、 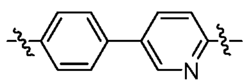 、 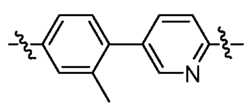 、

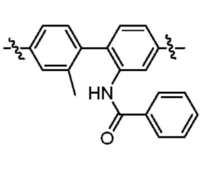 、 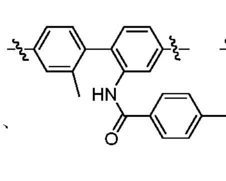 、 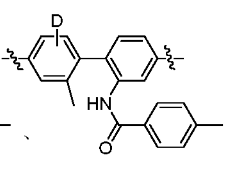 ;

wherein D is halogen.

**5.** The compound of claim 1, wherein in the Formula I, Z is substituted or unsubstituted $C_1$-$C_4$ alkyl or hydroxy.

**6.** The compound of claim 1, wherein in the Formula I, a structure of Z is

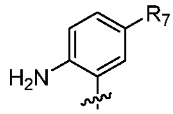

or hydroxyl; wherein $R_7$ is selected from any one of substituted or unsubstituted $C_1$-$C_{12}$ alkyl, substituted or unsubstituted $C_1$-$C_{12}$ heteroalkyl, substituted or unsubstituted $C_3$-$C_{12}$ cycloalkyl, substituted or unsubstituted $C_3$-$C_{12}$ heterocycloalkyl, substituted or unsubstituted $C_2$-$C_{12}$ alkenyl, substituted or unsubstituted $C_2$-$C_{12}$ heteroalkenyl, substituted or unsubstituted $C_3$-$C_{12}$ cycloalkenyl, substituted or unsubstituted $C_3$-$C_{12}$ heterocycloalkenyl, substituted or unsubstituted $C_2$-$C_{12}$ alkynyl, substituted or unsubstituted $C_2$-$C_{12}$ heteroalkynyl, substituted or unsubstituted $C_3$-$C_{12}$ cycloalkynyl, substituted or unsubstituted $C_3$-$C_{12}$ heterocycloalkynyl, substituted or unsubstituted aryl, substituted or unsubstituted heteroaryl, aryl ($C_1$-$C_{12}$) alkyl, heteroaryl ($C_1$-$C_{12}$) alkyl, $C_2$-$C_{12}$ alkenyl ($C_1$-$C_{12}$) alkyl, $C_2$-$C_{12}$ alkynyl ($C_1$-$C_{12}$) alkyl, hydroxyl, amino, nitro, sulfo, halogen and hydrogen atom.

**7.** The compound of claim 1, wherein in the Formula I, Y is a substituted or unsubstituted 5- or 6-membered nitrogen-containing heterocycloalkyl.

**8.** The compound of claim 7, wherein Y is selected from: any one of

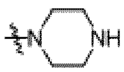, 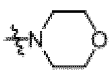, 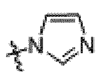, 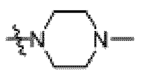, 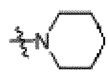.

**9.** The compound of claim 1, wherein the compound is selected from any one of

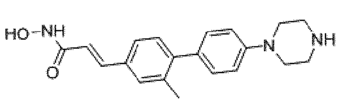, 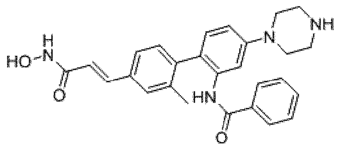, 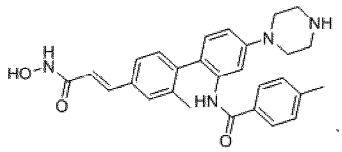,

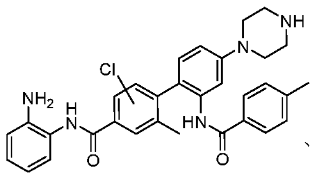, 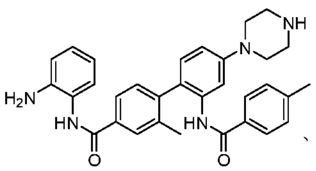, 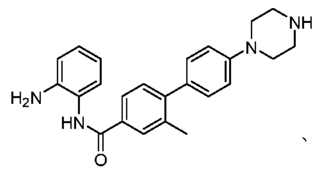,

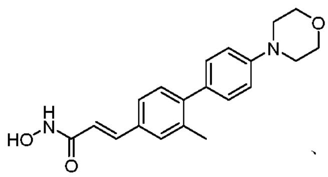, 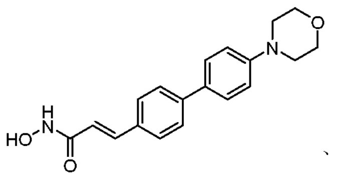, 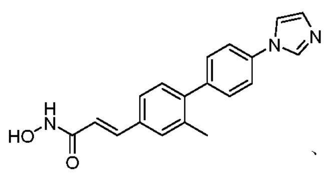,

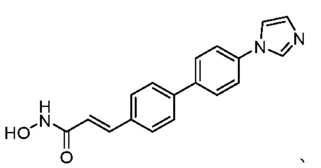, 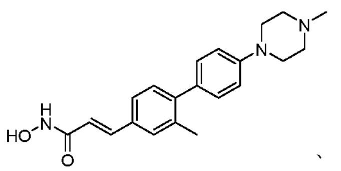, 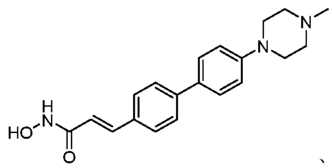,

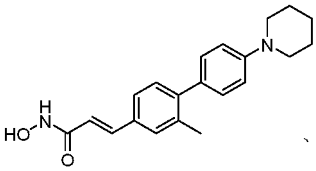,
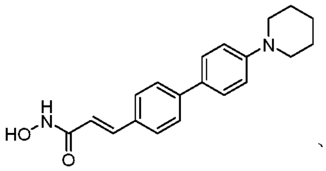,
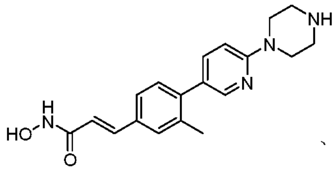,
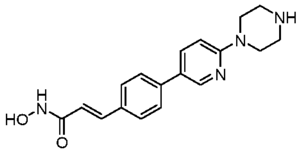.

**10.** A pharmaceutically acceptable salt of the compound of any one of claims 1-9.

**11.** A pharmaceutical composition comprising the compound of any one of claims 1-9 and a pharmaceutically acceptable carrier.

**12.** The pharmaceutical composition of claim 11, wherein the pharmaceutical composition further comprises an anticancer drug.

**13.** The pharmaceutical composition of claim 12, wherein the anticancer drug is an LSD1 inhibitor and/or an HDAC inhibitor.

**14.** Use of the compound of any one of claims 1-9 or a pharmaceutically acceptable salt thereof in preparation of a drug for the treatment and/or prevention of a tumor or cancer associated with LSD1 and/or HDAC.

**15.** The use of claim 14, wherein the tumor or cancer is selected from: at least one of brain cancer, glioblastoma, leukemia, Bristlecone syndrome, Cowden's Disease, cerebellar dysplastic gangliocytoma, breast cancer, inflammatory breast cancer, Wilm's tumor, Ewing's sarcoma, rhabdomyosarcoma, ependymoma, medulloblastoma, colon cancer, head and neck cancer, kidney cancer, lung cancer, liver cancer, melanoma, kidney cancer, ovarian cancer, pancreatic cancer, prostate cancer, sarcoma, osteosarcoma, giant cell tumor of the bone and thyroid.

**INTERNATIONAL SEARCH REPORT**

International application No. **PCT/CN2018/071317**

**A. CLASSIFICATION OF SUBJECT MATTER**

C07D 211/00(2006.01)i

According to International Patent Classification (IPC) or to both national classification and IPC

**B. FIELDS SEARCHED**

Minimum documentation searched (classification system followed by classification symbols)

C07D

Documentation searched other than minimum documentation to the extent that such documents are included in the fields searched

Electronic data base consulted during the international search (name of data base and, where practicable, search terms used)

CPRSABS, CNKI, VEN, CA, STN: 组蛋白, 北京大学深圳研究生院, 组蛋白去乙酰化酶, 联苯基, 癌症, 肿瘤, HDAC, histone deacetylase, histone, tumor, cancer, biphenyl

**C. DOCUMENTS CONSIDERED TO BE RELEVANT**

| Category* | Citation of document, with indication, where appropriate, of the relevant passages | Relevant to claim No. |
|---|---|---|
| X | CN 1425026 A (FUJISAWA PHARMACEUTICAL CO., LTD.) 18 June 2003 (2003-06-18) description, page 66, preparations 96-99 | 1-8 |
| A | Sabrina Dallavalle et al. "Design, Synthesis, and Evaluation of Biphenyl-4-yl-acrylohydroxamic Acid Derivatives as Histone Deacetylase (HDAC) Inhibitors" *European Journal of Medicinal Chemistry,* Vol. 44, 19 November 2008 (2008-11-19), pp. 1900-1912 | 1-15 |
| A | Raffaella Cincinelli et al. "Influence of the Adamantyl Moiety on the Activity of Biphenylacrylohydroxamic Acid-based HDAC Inhibitors" *European Journal of Medicinal Chemistry,* Vol. 79, 08 April 2014 (2014-04-08), pp. 251-259 | 1-15 |
| A | CN 101198586 A (SIGMA TAU INDUSTRIE FARMACEUTI) 11 June 2008 (2008-06-11) claims 1-19 | 1-15 |
| A | CN 101296897 A (GALDERMA RESEARCH & DEVELOPMENT) 29 October 2008 (2008-10-29) entire document | 1-15 |

☐ Further documents are listed in the continuation of Box C.

☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **24 June 2018** | **29 June 2018** |
| Name and mailing address of the ISA/CN<br>**State Intellectual Property Office of the P. R. China (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2018/071317**

| Patent document cited in search report | Publication date (day/month/year) | Patent family member(s) | Publication date (day/month/year) |
|---|---|---|---|
| CN 1425026 A | 18 June 2003 | CA 2400872 C | 16 November 2010 |
| | | MX PA02008171 A | 29 November 2002 |
| | | AT 293126 T | 15 April 2005 |
| | | RU 2224765 C1 | 27 February 2004 |
| | | PT 1259535 T | 30 June 2005 |
| | | DE 60110046 D1 | 19 May 2005 |
| | | ES 2236177 T3 | 16 July 2005 |
| | | IL 150904 D0 | 12 February 2003 |
| | | US 6884774 B2 | 26 April 2005 |
| | | PL 357875 A1 | 26 July 2004 |
| | | RU 2002125463 A | 20 February 2004 |
| | | OA 12180 A | 09 May 2006 |
| | | TW I250992 B | 11 March 2006 |
| | | PT 1259535 E | 30 June 2005 |
| | | EP 1259535 B1 | 13 April 2005 |
| | | NO 20023697 A | 14 October 2002 |
| | | WO 0160846 A1 | 23 August 2001 |
| | | CA 2400872 A1 | 23 August 2001 |
| | | JP 3906690 B2 | 18 April 2007 |
| | | CZ 20022844 A3 | 15 January 2003 |
| | | JP 2003523349 A | 05 August 2003 |
| | | NZ 520808 A | 26 March 2004 |
| | | KR 100437176 B1 | 25 June 2004 |
| | | AR 033666 A1 | 07 January 2004 |
| | | DE 60110046 T2 | 17 November 2005 |
| | | BR 0108792 A | 03 December 2002 |
| | | NO 20023697 D0 | 06 August 2002 |
| | | HU 0300066 A3 | 28 July 2004 |
| | | EP 1259535 A1 | 27 November 2002 |
| | | US 2003083238 A1 | 01 May 2003 |
| | | HU 0300066 A2 | 28 June 2003 |
| CN 101198586 A | 11 June 2008 | TW 200740728 A | 01 November 2007 |
| | | JP 2008546822 A | 25 December 2008 |
| | | EP 1896399 B1 | 17 November 2010 |
| | | SI 1896399 T1 | 31 August 2011 |
| | | US 7728039 B2 | 01 June 2010 |
| | | AR 057407 A1 | 05 December 2007 |
| | | AU 2006263915 A1 | 04 January 2007 |
| | | DK 1896399 T3 | 21 February 2011 |
| | | US 2008319082 A1 | 25 December 2008 |
| | | JP 5080462 B2 | 21 November 2012 |
| | | HR P20110106 T1 | 31 March 2011 |
| | | CA 2607207 A1 | 04 January 2007 |
| | | MX 2007014647 A | 11 February 2008 |
| | | CY 1111451 T1 | 05 August 2015 |
| | | KR 101288376 B1 | 22 July 2013 |
| | | KR 20080017028 A | 25 February 2008 |
| | | AU 2006263915 B2 | 01 December 2011 |
| | | TW I383969 B | 01 February 2013 |
| | | PT 1896399 E | 16 February 2011 |

Form PCT/ISA/210 (patent family annex) (January 2015)

**INTERNATIONAL SEARCH REPORT**
**Information on patent family members**

International application No.
**PCT/CN2018/071317**

| Patent document cited in search report | Publication date (day/month/year) | Patent family member(s) | Publication date (day/month/year) |
|---|---|---|---|
| | | BR PI0612492 A2 | 23 November 2010 |
| | | SI EP1896399 T1 | 31 August 2011 |
| | | WO 2007000383 A1 | 04 January 2007 |
| | | CA 2607207 C | 20 January 2015 |
| | | EP 1896399 A1 | 12 March 2008 |
| | | EA 200800156 A1 | 28 April 2008 |
| | | CN 102993051 A | 27 March 2013 |
| | | EA 012729 B1 | 30 December 2009 |
| | | AT 488493 T | 15 December 2010 |
| | | RS 51576 B | 31 August 2011 |
| | | DE 602006018298 D1 | 30 December 2010 |
| | | ES 2356129 T3 | 05 April 2011 |
| CN 101296897 A | 29 October 2008 | RU 2008120685 A | 10 December 2009 |
| | | JP 2009513620 A | 02 April 2009 |
| | | AR 058155 A1 | 23 January 2008 |
| | | US 2009012129 A1 | 08 January 2009 |
| | | WO 2007049158 A2 | 03 May 2007 |
| | | KR 20080061380 A | 02 July 2008 |
| | | WO 2007049158 A3 | 04 October 2007 |
| | | FR 2892412 A1 | 27 April 2007 |
| | | JP 5355089 B2 | 27 November 2013 |
| | | US 8383652 B2 | 26 February 2013 |
| | | BR PI0619326 A2 | 27 September 2011 |
| | | AU 2006307605 A1 | 03 May 2007 |
| | | EP 1943213 A2 | 16 July 2008 |
| | | CA 2625541 A1 | 03 May 2007 |
| | | FR 2892412 B1 | 16 May 2008 |

Form PCT/ISA/210 (patent family annex) (January 2015)